# EUROPEAN PATENT SPECIFICATION

(11) **EP 1 175 509 B1**
(45) Date of publication and mention of the grant of the patent: **20.09.2006**
(21) Application number: 00928358.1
(22) Date of filing: 26.04.2000
(51) Int. Cl.: C12Q 1/46, C12Q 1/34, G01N 33/543

(54) **IMMOBILIZED ENZYMES AS BIOSENSORS FOR CHEMICAL TOXINS**
IMMOBILISIERTE ENZYME ALS BIOSENSOREN FUR CHEMISCHE TOXINE
ENZYMES IMMOBILISEES SERVANT DE BIODETECTEURS DE TOXINES CHIMIQUES

(30) Priority: 26.04.1999 US 130989 P
(43) Date of publication of application: 30.01.2002
(73) Proprietor: U.S. Army Medical Research and Materiel Command, Fort Detrick, MD 21702-5012 (US)
(72) Inventor: GORDON, Richard, K., Potomac, MD 20854 (US); DOCTOR, Bhupendra, P., Potomac, MD 20854 (US)
(74) Representative: Sexton, Jane Helen
(86) International application number: PCT/US2000/011058
(87) International publication number: WO 2000/065081

(56) References cited:
- EP-A- 0 376 133
- WO-A-85/04424
- WO-A-94/05808
- DD-A- 301 391
- US-A- 4 411 989
- US-A- 4 525 704
- US-A- 4 826 759
- US-A- 5 292 669
- GORDON R K ET AL: "Organophosphate skin decontamination using immobilized enzymes." CHEMICO-BIOLOGICAL INTERACTIONS, (1999). PP. 119-120 (0) PP. 463-470. CODEN: CBINA8., XP000938329 Division of Biochemistry, Walter Reed Army Institute of Research, Washington, DC, 20307, USA.
- LEJEUNE ET AL: "Dramatically stabilized phosphotriesterase polymers for nerve agent degradation" BIOTECHNOL.BIOENGIN, vol. 54, no. 2, 1997, pages 105-114, XP002149622
- LEJEUNE KE ET AL: "Covalent linkage of mammalian cholinesterases within polyurethane foams." MED. DEF. BIOSCI. REV., PROC., (1996). VOL. 1, PP. 223-230. CODEN: 64UTAN., XP000938210 Center for Biotechnology and Bioengineering, Univ. of Pittsburgh, Pittsburgh
- MAXWELL ET AL: "Improvements in scavenger protection against organophosphorus agents by modification of cholinesterases" CHEM.BIOL.INTERACT., vol. 119-120, 1999, pages 419-428, XP000938330

## Description

### Technical Field

This invention relates generally to the field of detecting hazardous chemicals. More particularly, the present invention relates to methods, compositions, devices and kits thereof useful in the detection of chemical warfare agents and insecticides.

### Background of the Invention

Organophosphorus and organosulfur (OP refers to both types) compounds, are used extensively in insecticides and are highly toxic to many organisms including humans. Insecticide residues are found in soil and groundwater, and the detection of these residues is important for their elimination from the environment and to protect the health of both humans and animals. OP compounds are also used in nerve agents, such as sarin, phosphine, soman, and tabun, for chemical warfare purposes.

These agents are some of the most potent toxic agents and are specific inhibitors of acetylcholinesterase (AChE). The sequel to AChE poisoning is a cholinergic crisis in man; the clinical effects are directly related to acetylcholine accumulation. Nerve agents are classified into G agents (GD, soman; GB, sarin; and GA, tabun) and the V agents (VX). These agents differ in physical properties, for example, VX has a much lower vapor pressure than the G agents. However, the toxicity and main effects of the agents are very similar-inhibition of acetylcholinesterase and subsequent breakdown of the normal operation of the automatic and central nervous systems. Thus, detection of organophosphorous compounds is of paramount importance to prevent casualties due to OP exposure.

The need for the reliable determination of these cholinesterase inhibitors has led to the development of a number of sophisticated instrumental methods, mostly involving the use of gas and liquid chromatography and mass spectrometry. Also a number of liquid phase chemiluminescence procedures have been developed for the determination of inorganic and organic species mostly utilizing the luminol and peroxyoxalate reactions. *See* Robards K. and Worsfold P. J., (1992) Anal. Chem. Acta, 266:147.

These traditional methods are not practical for individual use as the methods are time consuming and complicated and the instruments utilized are expensive, non-portable and require high maintenance. Additionally, the measurement of nerve agents in mixtures with these traditional methods requires cumbersome extraction and manipulation procedures.

Thus, biosensors were developed as an alternative to the traditional gas and liquid chromatography and mass spectrometry technology. Generally, biosensors include those which are enzyme-based and bioaffinity-based. An enzymatic biosensor uses an enzymatic or metabolic process to detect a reaction product which occurs between an incoming substrate and an immobilized enzyme. A bioaffinity sensor relies on a biological binding event of a target substance.

The prior art biosensors are electronic devices which produce electronic signals as the result of biological interactions. These biosensors comprise a biological receptor linked to an electronic transducer in such a way that biochemical activity is converted into electrical activity. The electronic component of the biosensors measure voltage, amperage, wavelengths, temperature, or mass. *See* Lowe, C. R. (1984) Biosensors 1:3-16.

Biosensors are widely used to detect biological, pharmacological, or clinically important compounds. Generally, enzyme biosensors are selective, sensitive and specific. They are portable, simple and easy to use. Enzymatic biosensors can detect only those substances of interest and ignore all other environmental and biological interference.

Various cholinesterases (ChEs) biosensors have been described. These biosensors comprise ChEs non-covalently immobilized on a support. Cholinesterases have been immobilized on a full gamut of solid and gel supports such as glass, silica, ion-exchange resins, agarose, and nylon supports. Ideally, the preferred methods of immobilizing enzymes on solid supports have high coupling rates and the preferred biosensors retain enzymatic activity and maintain stability. However, biosensors which have non-covalently bound enzymes possess undesirable characteristics such as enzymatic instability at ambient and/or denaturing conditions, a propensity of the enzymes to leach from the surface to which it was non-covalently bound, and a short half-life in solution.

Generally, most methods which covalently bind enzymes to polymers utilize harsh or protein unfriendly conditions diminish enzymatic activity and stability. Although U.S. Patent No. 4,342,834 discloses a method of making isocyanate-based polyurethane foams wherein enzymes having varying degrees of activity are covalently linked, it does not disclose a material useful for the detection of OP compounds nor does it disclose a method of using the material for the detection of OP compounds.

Furthermore, it does not disclose a method of immobilizing enzymes on a porous support, whereby air induced shear forces are reduced which results in the retention of enzymatic activity.

Lejeune *et al*., Biotechnol Bioengin 54 (2), 105-114 (1997) describes stabilised phosphotriesterase polymers for nerve agent degradation. US Patent No. 4525704 describes an enzymatic toxic gas sensor having a plurality of parallel planar surfaces and a buffered electrolyte reservoir. The buffered electrolyte is conveyed by means of diffusion to a substrate to dissolve the substrate. The substrate diffuses to an immobilized enzyme where it is hydrolysed if the enzyme is active.

Lejeune *et al.,* Med Def. Biosci. Rev. Proc 1, 223-230 (1996) describes covalent linkage of mammalian cholinesterases within polyurethane foams for use in detoxification or decontamination of organophosphorus compounds from biological and other surfaces.

US Patent No. 4411989 describes methods and devices for detecting or monitoring or identifying substances and residual environmental pollutants using the discovering that spectra of an uninhibited enzyme can differ from spectra of the same enzyme which has been complexed with the agent pollutant. WO 85/04424 describes a detector kit for the indication of nerve gases and other cholinesterase inhibitors. WO 94/05808 describes a biosensor for detection of and distinguishing between cholinesterase inhibitors. DD301391A describes an enzymatic rapid detection material for detection of toxic chemicals.

### Summary of the Invention

In one embodiment the invention provides a biosensor for analyzing a sample for at least one organophosphorous and/or an organosulfur compound comprising at least one organophosphorus (OP) sensing enzyme and a measuring enzyme, which reacts with a product of OP sensing enzyme to form a detectable product, or a differential detection enzyme, which reacts with the organophosphorous or organosulfur compound to form a product which either now reacts with or does not react with the OP sensing enzyme, wherein the OP sensing enzyme is immobilized on or within a foam support by covalent bonds, wherein the OP sensing enzyme is selected from the group consisting of:
acetylcholinesterase (AChE), butyrylcholinesterase (BChE), and
pseudocholinesterase, the measuring enzyme is selected from choline oxidase or peroxidase and the differential detection enzyme is selected from triesterase, organophosphate hydrolase (OPH), laccase or paraoxonase and wherein the organophosphorous and/or organosulfur compounds are inhibitors of one or more of the OP sensing enzymes.

In a preferred embodiment, the biosensor is a material which comprises an enzyme that is covalently bound to a porous support, the immobilized enzyme exhibits enzymatic stability at extreme temperatures and/or denaturing conditions, and similar kinetic characteristics of the soluble form. The enzyme does not leach from the porous support and the material retains enzymatic activity after prolonged storage.

In another embodiment, a plurality of materials comprising a porous support wherein a plurality of enzymes is immobilized upon or within which the plurality of materials is suitable for use as a differential biosensor having multiple zones for detecting the presence of specific OP compounds. In a preferred embodiment, the dimensions of the biosensor are in the range from about 6.35 x 6.35 x 0.79mm (0.25" x 0.25" x 0.003125") to about 152 x 102 x 6.35mm (6" x 4" x 0.25").

Yet another embodiment of the invention provides a method of making a biosensor as defined above for detecting and measuring a hazardous compound said chemical being an organophosphorus and/or an organosulfur compound which is an inhibitor of one or more of AChE, BChE and pseudocholinesterase, said method comprising immobilizing by covalently binding a plurality of enzymes or a cross-linked enzyme complex on, within or encapsulated in a porous support wherein the plurality of enzymes or the cross-linked enzyme complex comprises at least one OP sensing enzyme and a measuring enzyme, which reacts with a product of OP sensing enzyme to form a detectable product, or a differential detection enzyme, which reacts with the organophosphorous or organosulfur compound to form a product which either now reacts with or does not react with the OP sensing enzyme, wherin the OP sensing enzyme is an enzyme selected from the group consisting of: acetylcholinesterase (AChE), butyrylcholinesterase (BChE), and pseudocholinesterase, the measuring enzyme is selected from choline oxidase or peroxidase and the differential detection enzyme is selected from triesterase, organophosphate hydrolase (OPH), laccase or paraoxonase.

Synthesis of the porous support involves the use of prepolymer such as polyether and a surfactant such as P-65. In a preferred embodiment, the method of making the material reduces air induced shear forces or shear stress which reduce enzymatic activity. During synthesis of the material by prior art methods, for example a mixing drill, the enzymes utilized are subjected to fluid forces or shear stress. Use of a device that gently folds the components into one another greatly reduces these fluid forces or shear stress, and is the preferred device for enzymes, specifically enzymes that are sensitive to the high shear forces of the drill mixing device. The low shear mixing device more than doubles the resultant AChE or BChE immobilized enzyme activity when compared to an identical mixture prepared with the high shear device. Additionally, use of additives such as surface-acting polymers, *e.g*. P-65, or low concentrations of glycerol protects against enzyme denaturation induced by shear forces.

As disclosed, herein, one of ordinary skill in the art may synthesize a variety of porous supports with the various prepolymers and surfactants available.

When immobilized, the enzymes of the biosensors are stable under extreme temperatures and/or denaturing conditions. These enzymes include cholinesterases, choline oxidase, hydrogen peroxidase, organophosphate hydrolase, phosphotriesterase, laccase and derivatives thereof.

The biosensors are suitable for detecting and quantifying OP chemicals in gases and liquids and on solids. For example, the biosensor may be used to detect OP compounds in water and/or soil. The biosensor may be used to detect OP compounds on natural, synthetic and/or biological surfaces, such as wood, plastic and skin. By using mammalian cholinesterases such as FBS-AChE or Eq-BChE rather than Eel Cholinesterase as is found in the M272 ticket (currently used to detect organophosphate compounds; available from Truetech, Inc.), the immobilized sensor will display the same sensitivity to OPs that mankind is suseptible to now, or any new or novel OPs that might be produced in the future against mankind.

In one embodiment, the biosensor comprises at least one indicator to indicate the presence of an OP compound by fluorescent, chromogenic or chemiluminescent determination.

In another embodiment the biosensor comprises a carbon electrode for the detection of an OP compound by determining cholinesterase activity.

In another embodiment, the amount and source of the various OP compounds may be determined.

Yet another embodiment relates to kits. Kits may include, along with the biosensor, the indicators for both quantitative or qualitative detection of OP compounds and means for transmitting results to a central collection point, e.g. computer, satellite uplinks, radio relays, handheld battery operated measuring devices, etc. For example, one may quantitatively analyze the OP compounds by using a handheld battery operated measuring devices and interfacing with a computer to calculate reaction rates which rates may be relayed to a central collection point. These kits may also contain instructions, solutions and compositions needed for operation. The compositions and solutions may be placed in containers, test tubes, etc.

Other embodiments include the methods of using the instant biosensors for the quantitative or qualitative determination of hazardous compounds such as OP compounds.

### Description of the Drawings

This invention is further understood by reference to the drawings wherein:

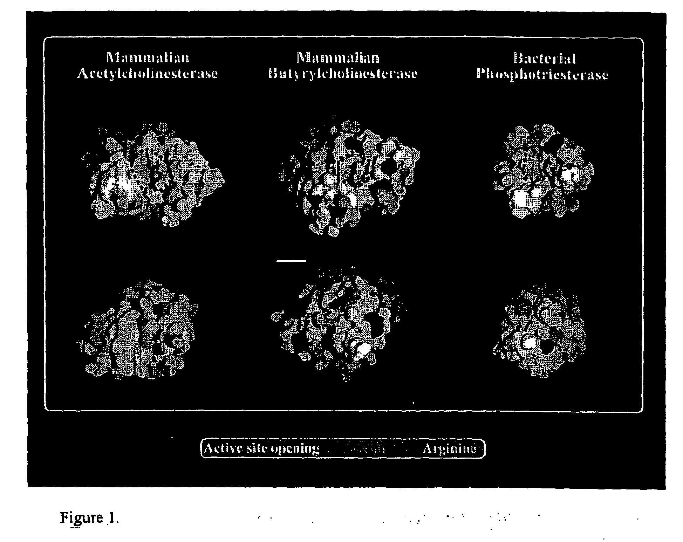

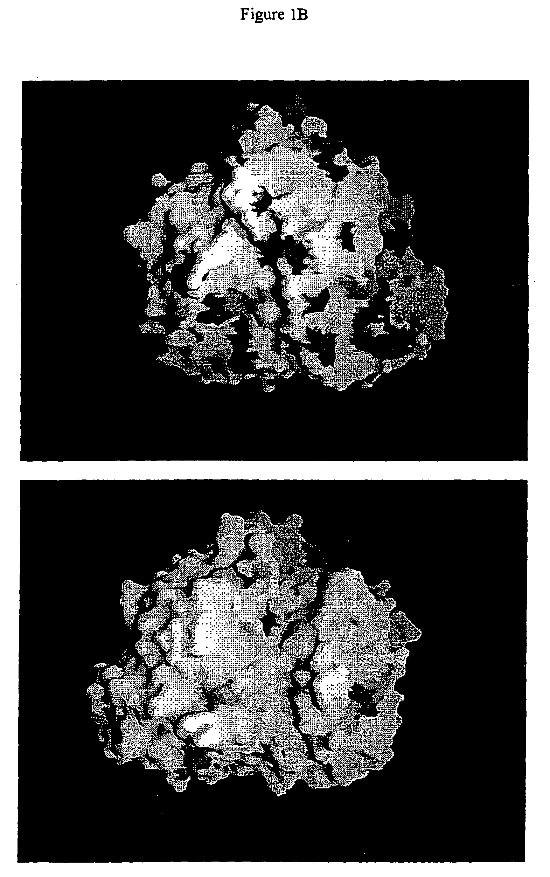

Figure 1A illustrates the modeled surfaces of acetylcholinesterase, butyrylcholinesterase and phosphotriesterase. The top row shows a view of the front of the enzymes with the lip of the active site gorge outlined with a dotted line in the center. The bottom row shows the backside of the enzymes (180° rotation). The Lysine and Arginine residues on the surface, which are potential coupling sites to the polymer, are shaded dark in both the top and bottom row. Figure 1B illustrates a model of the surface of laccase with available residues to couple covalently to the prepolymer (top, front of enzyme; bottom, backside of enzyme).
Figure 2 shows a cured material.
Figure 3 schematically illustrates the specific reaction of the enzymes with prepolymer.
Figure 4 shows the linear correlation between the amount of BChE added to the prepolymer during synthesis of the material and the amount of BChE in the final material (sponge).
Figure 5 shows the increasing amounts of BSA added during synthesis to a constant amount of AChE and TDI polymer.
Figure 6 illustrates that the materials, BChE-sponge, maintained enzymatic stability for more than 3 years at 4°C and up to 7 months at 25°C and up to 4 months at 45°C. Similar results were obtained with AChE-sponge.
Figure 7 shows that the material, AChE-sponge, maintained enzymatic activity after consecutive washes. An AChE-sponge was alternately washed with phosphate buffer and assayed for activity. This procedure was carried out for three days. Similar results were observed for BChE-sponge.
Figure 8 shows a substrate concentration dependent curve for soluble and polyurethane coupled AChE.
Figure 9 illustrates the pH range of soluble and immobilized AChE.
Figure 10 shows the relative activities of co-immobilized ChEs and OP hydrolases (OPHs).
Figure 11A shows a version of a manual mixing gun and Figure 11B shows a disposable mixing stator. Complete mixing of the enzyme in aqueous solution and the viscous prepolymer is accomplished in the stator. The product shown here for illustrative purposes is green, while the two starting components are yellow and blue.
Figure 12 shows a small material comprising AChE immobilized to the polyurethane. The material is a small sponge about 9.5mm (3/8") in diameter.
Figure 13A schematically illustrates alternate schemes for detecting ChE activity. Figure 13A shows a variety of possible detection methods, such as qualitative colorimetric changes, chemiluminescent for a dark environment, and additional amplification by coupling the ChE reaction to choline oxidase.
Figure 13B shows Amplex Red visual and fluorimetric pathway.
Figure 13C shows AChE and choline oxidase biosensor reaction to the OP MEPQ.
Figure 13D shows chemiluminesence of AChE/choline oxidase sensor.
Figure 14 is a model of a carbon electrode with immobilized ChE.
Figure 15 illustrates how high fluoride ion concentration F reverses the reaction between an OP compound and ChE. This reaction will permit the determination of the type of OP bound to the badge.
Figure 16 illustrates reactivation of alkyl phosphorylated ChE with oxime.
Figure 16A illustrates the enzyme activity of immobilized FBS-AChE. Figure 16B illustrates the enzyme activity of immobilized Eq-BChE.
Figure 17 represents inhibition of foam-immobilized FBS-AChE by DFP.
Figure 18 represents inhibition of foam-immobilized Eq-BChE by DFP.
Figure 19A shows the pH profile of soluble and immobilized choline oxidase. Figure 19B shows the Kₘ of the soluble and immobilized forms (sponge) of choline oxidase, ie. the substrate concentration dependent curve for soluble and polyurethane coupled choline oxidase.

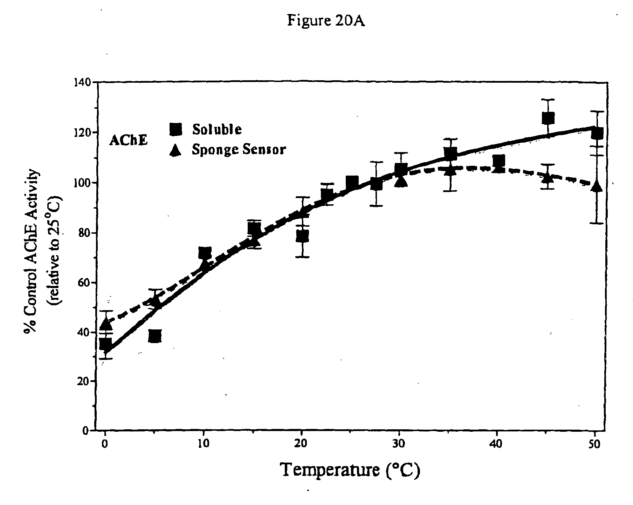

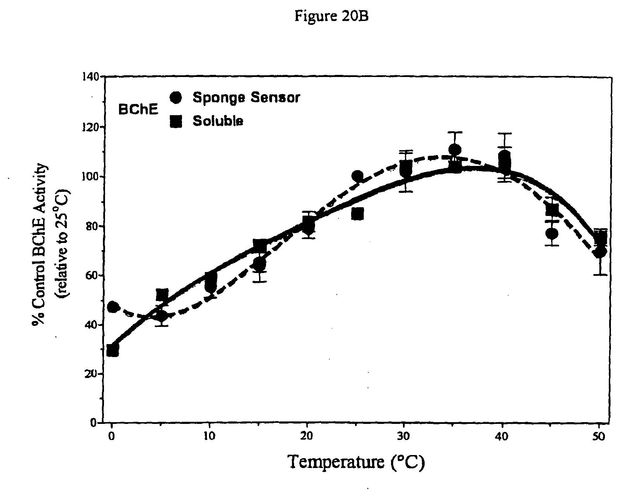

Figure 20A shows the temperature profile of Immobilized and Soluble AChE. Figure 20B shows the temperature profile of Immobilized and Soluble BChE.

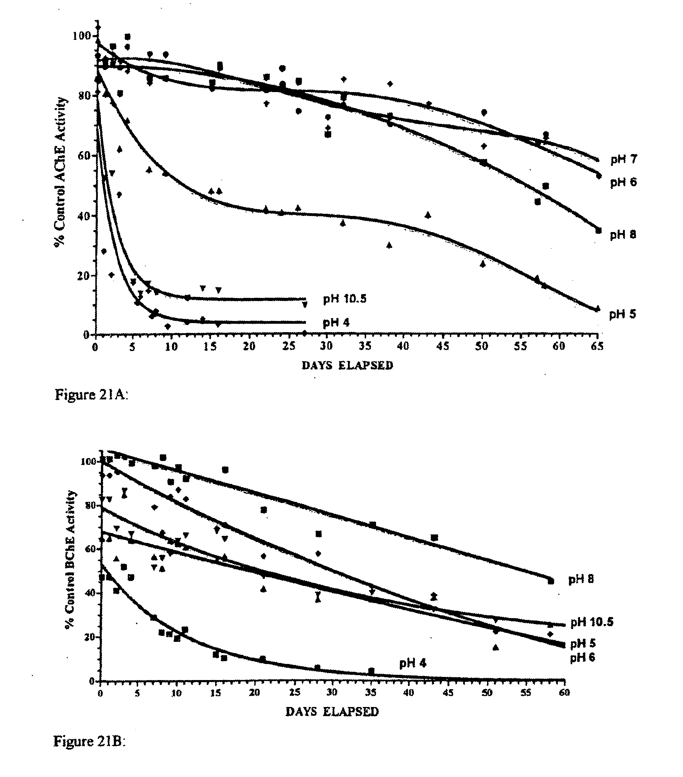

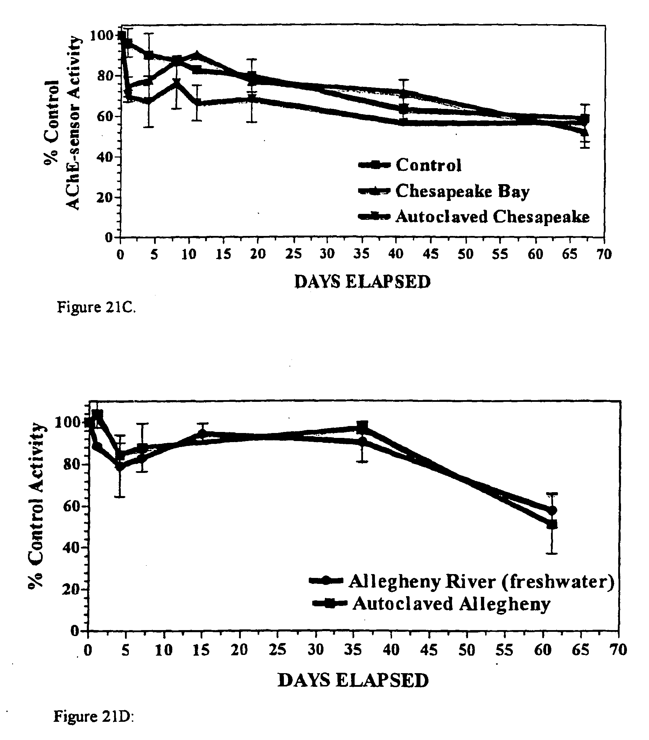

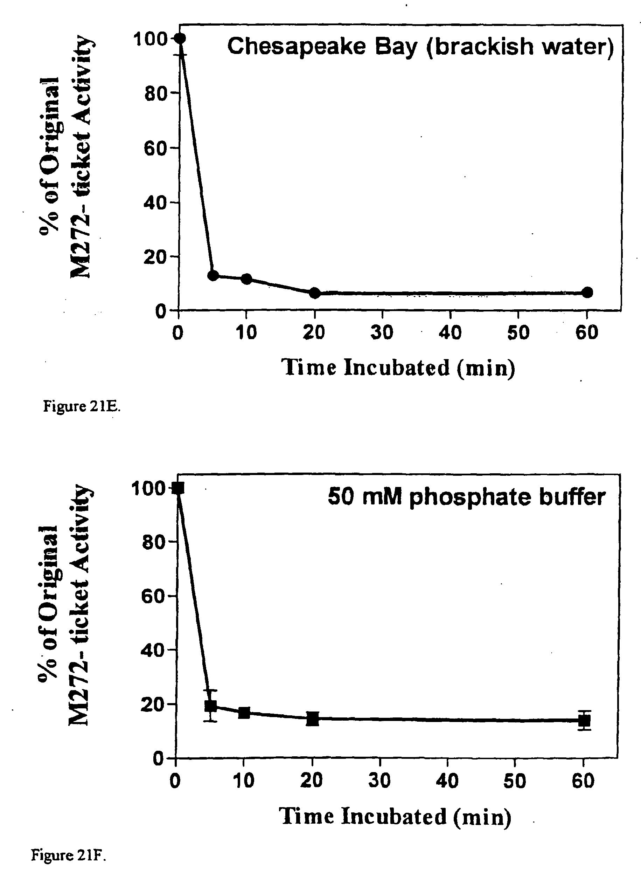

Figure 21A shows AChE-sensor activities after continuous incubation at 25°C at different pHs. Figure 21B shows BChE-sensor activities after continuous incubation at 25°C at Different pHs. Figure 21C shows AChE-sensor activity after continuous exposure to Chesapeake Bay (Brackish) Water at 25°C. Figure 21D shows AChE-sensor activity after continuous exposure to Allegheny River (Fresh) Water at 25°C. Figure 21E shows sensitivity of M272 ticket to aqueous conditions (Chesapeake Bay brackish water). Figure 21F shows sensitivity of M272 ticket to aqueous conditions (50 mM phosphate buffer, pH 8.0).

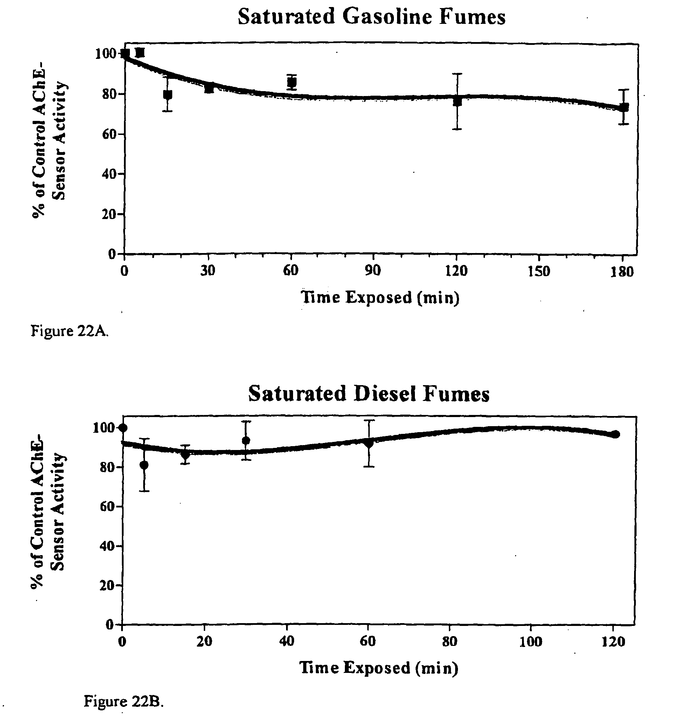

Figure 22A shows AChE sensors are resistant to continuous exposure to saturated gasoline fumes and thus do not yield false positives. Figure 22B shows AChE sensors are resistant to continuous exposure to saturated diesel fumes and thus do not yield false positives.
Figure 23A shows dose-dependent inhibition of immobilized AChE sensor and soluble AChE to the pesticide dichlorophos. Figure 23B shows dose-dependent inhibition of Immobilized AChE (sensor) and soluble AChE to the organophosphate soman (GD).

### Detailed Description of the Invention

Enzymes have been incorporated in hypo-based urethane foam during polymer synthesis. See U.S. Patent No. 4,342,834. Hypoprepolymer is synthesized from a reaction of polyether (or polyester) polyol with isocyanates in the presence of cross-linking agents. *See* Havens, P.L., *et al., Ind Eng Chem Res* (1993) 32:2254-2258; U.S. Patent No. 4,137,200; LeJeune, K.E., *et al., Biotechnology and Bioengineering* (1999) 20;62(6):659-665. Synthesis is initiated by bringing water molecules into contact with isocyanate groups present within the polyurethane prepolymer.

A two-step procedure occurs from this point. Isocyanates react with water to form an unstable carbonic acid, which in turn degrades to an amine yielding CO₂ that gives the porous support lift and enables it to rise. The amines readily react with isocyanate groups, leading to production of urea type linkages. Since the enzyme contains multiple functional groups, such as amines and hydroxyls that can react with isocyanates, the enzyme becomes an integral part of the porous support during synthesis. Significant quantities of enzyme can link to the porous support without disrupting the progress of polymer synthesis. The reaction occurring during the polymer synthesis is shown below.
1. CO₂ Evolution: 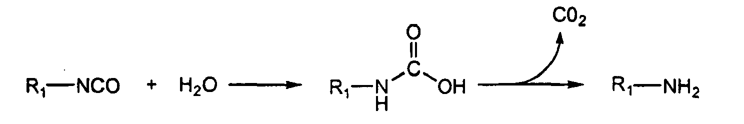
2. Urea Linkage: 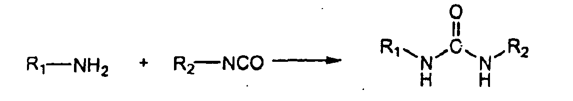
3. Amine Group Enzyme Immobilization: 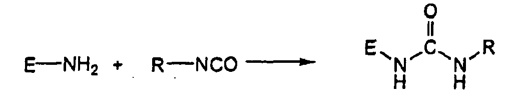
4. Hydroxyl Group Enzyme Immobilization:

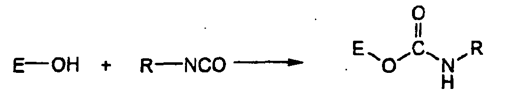

The following list of enzymes (the enzymes can be from a variety of sources, e.g. cell-free, recombinant, etc.) and chemicals are examples of those suitable for use in the instant invention:

### OP Sensing Enzymes

Acetylcholinesterase (AChE);
Butyrylcholinesterase (BChE);
Pseudocholinesterase;

### Measuring Enzymes

Choline oxidase;
Peroxidase;

### OP Differential Detection

Organophosphate hydrolases (OPH);
Phosphotriesterase;
*Pseudomonas diminuta* bacterial OPH (paraoxonase);
Laccases (plus mediators and/or cofactors of laccases);

### Other reagents

ABTS Trade Mark and analogs thereof;
Diisopropyl fluorophosphate (DFP);
7-(methoxyphosphinyloxy)-1-methylquinolium iodide (MEPQ);
Acetylthiocholine iodide (ATC);
S-butyrylthiocholine iodide (BTC);
5,5'-dithio-bis(2-nitrobenzoic acid) (DTNB);
N,N'-trimethylene bis(pyridinium-4-aldoxime) dibromide (TMB4); and
1-(2-hydroxyiminomethyl-1-pyridinium)-1-(4-carboxyaminopyridinium)-dimethylether hydrochloride (HI-6).

In general, the simplest biosensor for OP compounds comprises a material, having ChEs immobilized upon or within a porous support, secured upon a carrier such as plastic, glass, cloth, nylon, rubber, etc. Detection of OP compounds may be qualitatively determined by color. To test for OP compounds, biosensor is exposed to the sample to be tested. Since the inhibition of the immobilized ChE by an OP compound is substantially similar to that observed for the soluble form, only a short duration of exposure to the sample is required. *See* Table 1.

| Table 1. Time-Dependent Inhibition of ChEs by MEPQ | | |
|---|---|---|
| ChE | Enzyme Form | Bimolecular rate constant (M⁻¹ min⁻¹) ± SD |
| FBS-AChE | soluble | 1.59 ± 0.52 x 10⁸ |
| | coupled to sponge | 1.00 ± 0.28 x 10⁸ |
| | | |
| Equine-BChE | soluble | 4.15 ± 0.78 x 10⁷ |
| | coupled to sponge | 4.21 ± 2.00 x 10⁷ |

The material may be washed, squeezed or purified otherwise in order to remove compounds and/or compositions which may cause interference since the immobilized enzyme does not leach out and OP compound is irreversibly bound to the immobilized enzyme. A substrate may be embedded on or in the porous support or applied to the material after exposure to the sample wherein the substrate remains soluble or mobile. An appropriate buffer may be applied to the material. A change in color or luminescence of the material indicates the presence of an OP compound.

These biosensors may be disposed after a single use or may be reused. By reusing the biosensor, the material accumulates the OP compound and thereby indicates the cumulative amount of an OP compound over a defined time period. The biosensor may be regenerated using a reagent to displace the OP compounds, e.g. flouride salts. Accuracy of the biosensor is assured if it is recalibrated prior to use.

These qualitative tests for the presence of OP compounds do not require an external source of energy or other equipment. However, the biosensor may comprise a carbon electrode for the detection of an OP compound by determining cholinesterase activity as indicated by H₂O₂. In this embodiment, the material, wherein the ChE is immobilized upon or within, is secured on the carbon electrode. In the instant invention, the biosensors may be stored for long periods of time at extreme temperatures and may also be used repeatedly.

A few examples of biosensor include test strips, badges and patches.

The following examples are intended to illustrate the invention.

### Example 1

### Determination of Possible Enzyme Interference

As polyether prepolymer derived from tolyl diisocyanate (TDI), reacts most favorably with free aliphatic amines such as lysine and arginine present on the surface of the ChEs (or any protein) to become a permanent cross-linked part of the material, computer aided molecular modeling of the enzymes was performed to highlight the available amino groups on the surface of each enzyme, and to determine whether the coupling of these groups to a porous support would interfere with enzymatic function. This may be performed on every enzyme for which its crystal structure is known, or enzymes which may be modeled by homology.

Figure 1A illustrates the modeled surfaces of acetylcholinesterase, butyrycholinesterase and phosphotriesterase and shows the lysine and arginine residues on the surface of the ChEs which are available for coupling to the prepolymer. This was generated by Insight II, molecular modeling software, by Biosym Technologies. Based on the molecular modeling, there are at least one lysine and 29 arginine water-accessible residues on the surface of FBS-AChE to couple to the porous support, while 26 lysine and 26 arginine residues were modeled for equine-BChE. The majority of the lysine and arginine residues were found on the backside of the ChEs, and only a few are found on the side of the enzyme where the catalytic site gorge is located. The rim and the catalytic site gorge opening of both AChE and BChE appeared to be essentially devoid of lysine and arginine. Therefore, coupling these enzymes to the porous support should have minimal effect on the entrance of substrate, inhibitors such as OPs, or reactivators such as oximes which includes mono-disquartemary oximes, release of products of catalysis to and from the active site, and the kinetic rates of the enzymes. Similarly, a model of the surface of laccase (Figure 1 B) is shown with available residues to couple covalently to the prepolymer.

### Example 2

### Synthesis of an Enzyme Bound Polyurethane Material

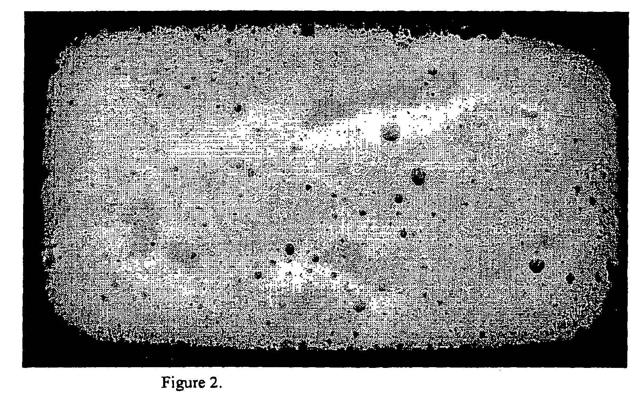

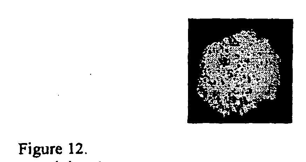

A typical synthesis of the material comprises mixing enzymes in phosphate buffer containing 1% (final concentration) surfactant with prepolymer. Polyether prepolymer derived from tolyl diisocyanate (TDI), Hypol Trade Mark prepolymer TDI 3000 (Hampshire Chemical, Lexington, MA), and Pluronic Trade Mark P-65 surfactant (BASF Specialty Chemicals, Parsippany, NJ) were used. The 2-phase system is mixed and placed into a suitable mold and left to cure. Figure 2 shows a cured material which comprises a sponge-like porous support. Figure 12 shows a small material comprising immobilized AChE.

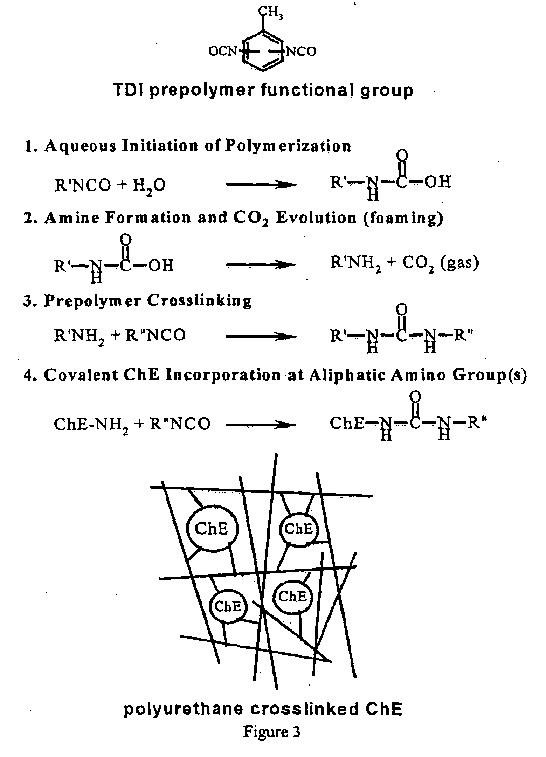

Figure 3 schematically illustrates the specific reaction of the enzymes with prepolymer. Synthesis begins when H₂O molecules react with the isocyanate groups present within the polyurethane prepolymer. Isocyanate reacts with the water to form an unstable carbonic acid, which degrades to an amine yielding CO₂. The CO₂ causes the polymer to rise and become porous, and simultaneously the amines readily react with the isocyanate groups leading to urea linkages.

Since the ChE contains amines that are on the surface and available to react with the isocyanate groups, they can become an integral part of the polyurethane support during synthesis. There is no significant entrapment of the enzyme in the material as found with cyclodextrins, or physical adsorption of the enzymes, as observed with activated carbon. The inclusion of a surfactant such as Pluronic Trade Mark P-65 at about 1% final concentration controls the final structure and absorption potential of the material.

To create a material comprising a porous polyurethane support, approximately 30 mL of 50 mM phosphate buffer, pH 8.0, containing P-65 surfactant buffer, was placed in a 600 mL plastic beaker. 3 to 5 mL of either purified FBS-AChE (7500 units) or purified Eq-BChE (5000 units) was added, followed by approximately 0.04 kg (40 gm) of Hypo 3000 prepolymer (tolyl diisocyanate). The two-phase system was mixed and the material was allowed to expand for 10 min, extruded from the container. The material was washed thoroughly with 50 mM phosphate buffer, pH 8.0, dried and stored in a zippered bag at 4°C for future use.

### Example 3

### Characteristics of Synthesized Material

Approximately 20-90% of the enzymes were covalently linked to the porous support through free amino- or hydroxyl groups. This was determined by the presence of enzyme in first and second washes of the material.

Since the enzymes can be attached at multiple points, they become a part of the cross-linked polymer support. The cross-linked polymer support imparts considerable stability to the bound enzymes. A large quantity of enzyme can be incorporated into a small polyurethane support, thereby rendering the cross-linked polymer support a highly stable and sensitive material for detection of OP compounds.

### A. Enzymatic Activity

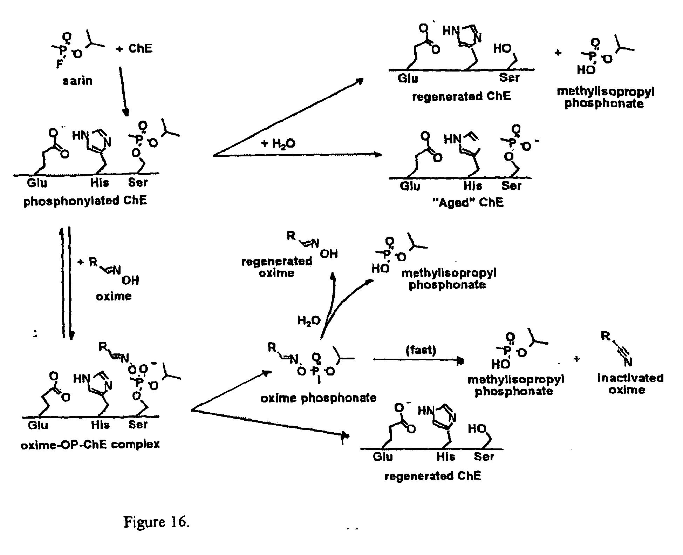

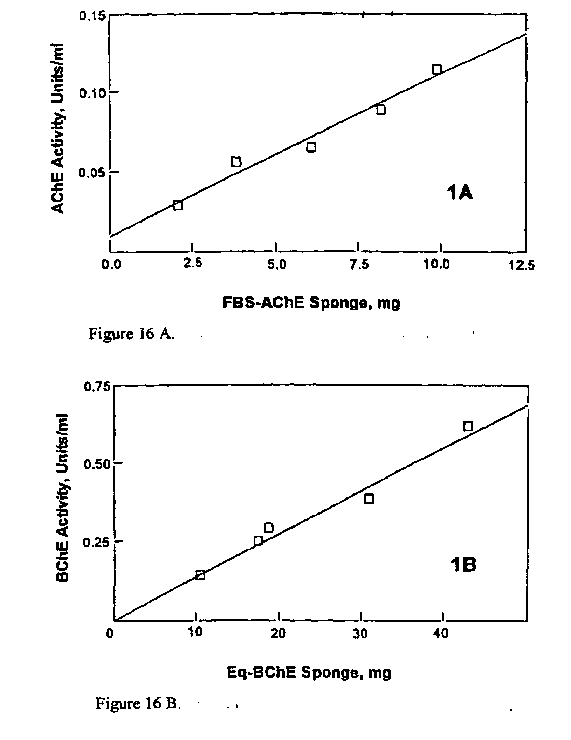

Five samples of materials containing FBS-AChE and five samples of materials containing Eq-BChE, ranging in weight from 1 to 40 mg, were suspended in 2.8 mL of 50 mM phosphate buffer, pH 8.0, and assayed using the method of Ellman. *See* Ellman, G.L., *et al.,* (1961) *Biochem Pharmacol.* 7:88-95. A linear correlation was found between the weight of the sponge and enzyme activity for both FBS-AChE and Eq-BChE immobilizations. *See* Figs. 16 A and B. The linear correlation between the weight of the material and enzyme activity indicates a uniform immobilization of AChE or BChE throughout the material.

The material was washed with either 50 mM phosphate buffer, distilled water, or 10 mM ammonium bicarbonate without affecting substrate hydrolysis. Therefore, the mixing of prepolymer, surfactant, and enzyme *in situ* at 22°C yields a useful and effective material retaining about 50% of the original activity of soluble ChE.

### B. Protein Loading Capacity

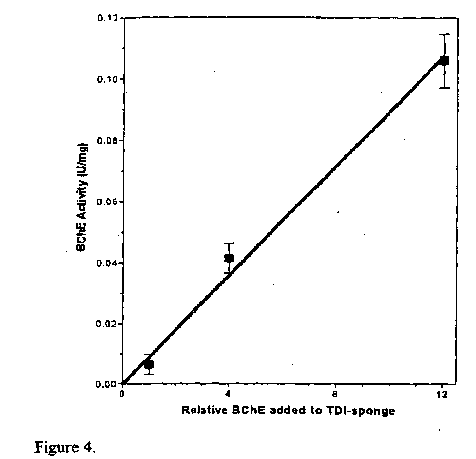

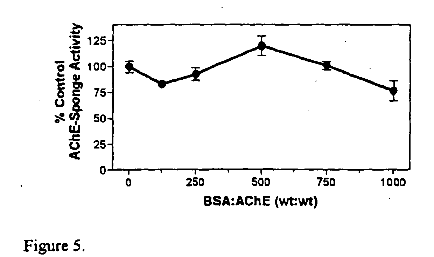

The material has a significantly higher loading capacity for ChEs such as BChE or AChE. The final activity of the BChE immobilized in the material could be increased by adding larger quantities of enzyme during synthesis. *See* Figure 4. When nonspecific protein (bovine serum albumin, BSA) was added to a constant amount of purified AChE, there was no reduction in ChE activity. *See* Figure 5. Thus, higher potency materials may be synthesized with additional proteins, enzymes, and other ChEs. Additionally, materials effective for detecting a diverse array of OP compounds may be readily synthesized by with combinations of *multiple* enzymes or a plurality of enzymes.

### C. Enzymatic Stability

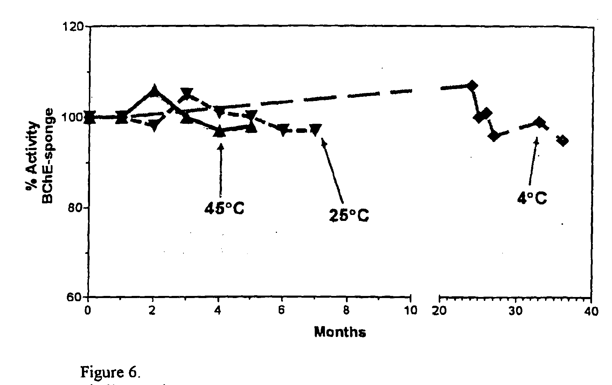

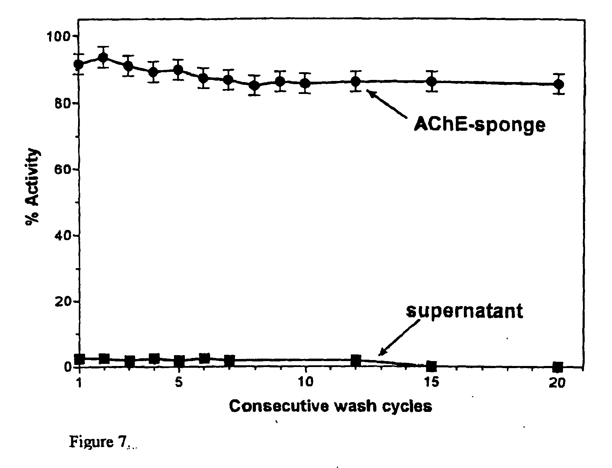

As illustrated by Figure 6, the immobilized ChE and OP hydrolase maintained enzymatic stability for more than 3 years at 4°C, up to 7 months at 25°C and up to 4 months at 45°C, respectively. If the material is frozen in liquid nitrogen, most of the original activity remains. TDI imparts remarkable stability to the immobilized ChE; about 50% of the original activity of the immobilied AchE and 20% of the activity of the immobilized BChE remained after 16 hours at 80°C, conditions under which the soluble enzymes would exhibit no activity. The ChE materials can be exhaustively dried under vacuum at 22°C and then rehydrated without loss of enzyme activity. When AChE or BChE materials were exhaustively washed and assayed for activity, the wash and assay cycle repeated more than twenty times over three days, no decrease in activity occurred. *See* Figure 7. This indicates that the material may be used repeatedly.

These results also demonstrate that the ChEs are covalently cross-linked in the porous support and that the ChEs will not leach out to skin, water, or equipment. Therefore, once the immobilized enzymes bind an OP compound the OP is removed from the surface requiring decontamination.

### D. Kinetic Constants

The number of active sites of either the immobilized or soluble ChEs was determined by titration with the organophosphorous compound MEPQ, 7-(methylethoxyphosphinyloxy)-1-methylquinolinium iodide. The bimolecular rate constants for the inhibition of AChE material and BChE material and the respective soluble enzymes by MEPQ at 25°C showed that there was no significant difference between the soluble and covalently bound enzymes. *See* Table 1. These results demonstrate that the immobilized and soluble forms of ChEs interact with the OP compounds similarly. Therefore, enzymatic activity assays which are generally available and known in the art may be used.

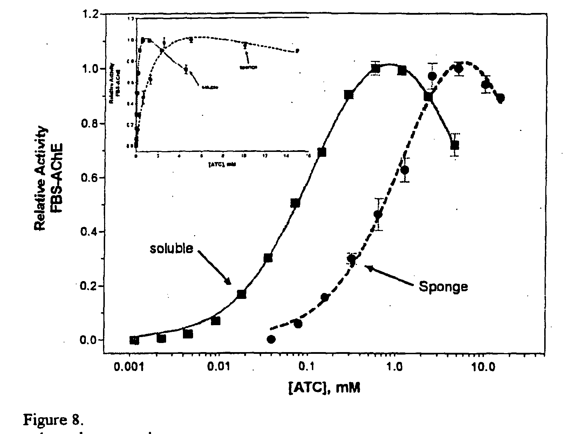

An initial rates method using a modified Ellman's assay was used to determine the parameters Kₘ, *k*_{*cat*}*, and k*_{*cat*}/Kₘ for immobilized and soluble AChE and BChE. The number of active sites of either the coupled or soluble ChEs was determined by titration with MEPQ: As shown in Table 2 and Figure 8 for AChE, the Kₘ values for the immobilized ChEs were about 10-fold greater than the corresponding soluble enzymes, and the *k*_{*cat*} values were less dramatically affected. The combined effects on affinity for substrate and *k*_{*cat*} resulted in approximately a 20 to 50-fold decrease in acylation (*k*_{*cat*}/Kₘ)*.* Interestingly, while soluble BChE lacked substrate inhibition, immobilized BChE yielded substrate inhibition. These results suggest that covalent binding of surface residues of ChEs to the porous support changed some properties of the active site region of the bound enzymes directly or indirectly.

| **Table 2. Kinetic parameters for soluble and polyurethane coupled ChEs.** | | | | | | | |
|---|---|---|---|---|---|---|---|
| Enzyme | Form | Substrate inhibition | Kₘ (mM) | Kₛₛ (mM) | B | K_{cat} (min⁻¹) | K_{cat}/Kₘ (M⁻¹min⁻¹) |
| FBS-AChE | Soluble | yes | 0.119 | 18 | - | 2.8 x 10⁵ | 2.5 x 10⁹ |
| | immobilized | yes | 1.090 | 22 | - | 5.9 x 10⁴ | 5.4 x 10⁷ |
| Equine-BChE | Soluble | no | 0.127 | 1.5 | 1.8 | 3.1 x 10⁴ | 2.4 x 10⁸ |
| | immobilized | yes | 1.200 | 16 | - | 1.8 x 10⁴ | 1.5 x 10⁷ |
| Determined in 50 mM phosphate, pH 8 at 25°C using an initial rates method. | | | | | | | |
| Calculated from Vₘₐₓ and the active site concentration of ChE that was determined by MEPQ titration. | | | | | | | |
| Values were calculated² using modified Haldane equations, and the special case where b=0. The best fit between the two was determined using an F test, where significance was defined as p<0.05. | | | | | | | |

### Kₘ Determination of immobilized and soluble choline oxidase:

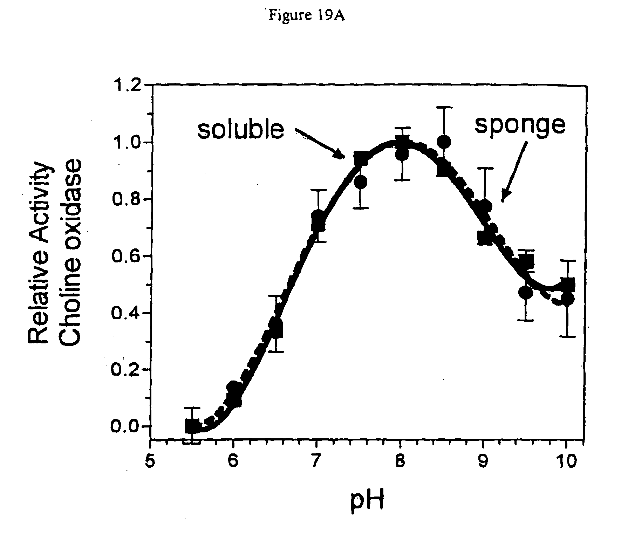

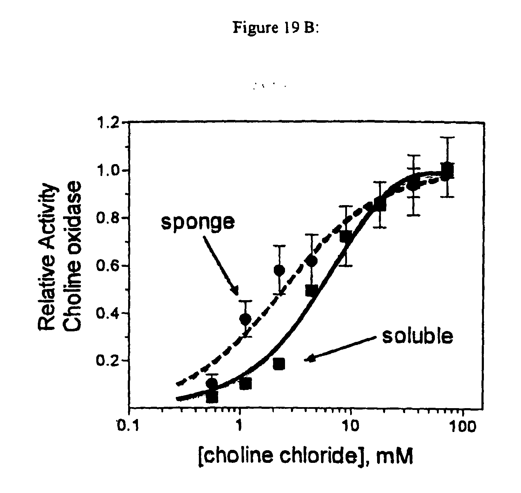

The Kₘ of the soluble and immobilized forms (sponge) of choline oxidase are observed to be similar since there is little shift in the substrate curve, as shown by Figure B, indicating that this enzyme is not only very suited to immobilization, but also for co-immobilization with the cholinesterases. The observed Kₘ for soluble and sponge are 2.5 and 6.7 mM, respectively. See Figure 19B.

### E. pH of Soluble and Immobilized Enzymes

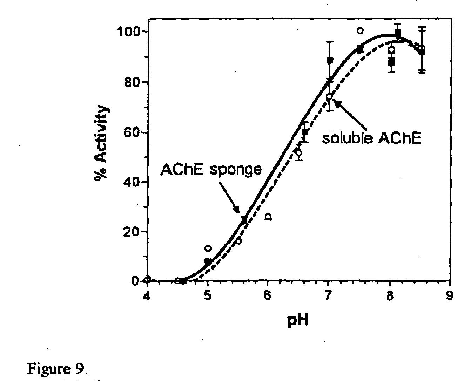

The pH profiles of immobilized and soluble AChE are identical and the enzymes exhibit activity throughout the broad pH range of 7-8.5. *See* Figure 9. Since the pH profiles of soluble cholinesterases, choline oxidase and OP hydrolases have optimal activities in this same pH range, the materials may be optimized and diversified by employing a plurality of these multiple enzymes immobilized on or within a porous support.

### pH of soluble and Immobilized Enzymes as more supporting evidence

Figure 19A: The pH profile of soluble and immobilized choline oxidase. Compare with Figure 9, the pH profile of soluble and immobilized acetylcholinesterase.

### F. Temperature Dependent Activity of Soluble Cholinesterases and Sensor (Immobilized) Cholinesterases

The sensors containing immobilized AChE or BChE exhibited almost identical temperature dependent activity when compared to their soluble counterparts. However, as shown in Figure 6, the immobilized enzymes are more resistant to the denaturing conditions of elevated temperatures for extended periods, while the soluble enzymes are not. The immobilized enzymes are also resistant to freezing in liquid nitrogen. These profiles indicate that at cold temperatures, the sensors could be warmed by body heat or an external source to increase the reaction rates.

### Example 4

### Immobilization of a Plurality of Multiple Enzymes

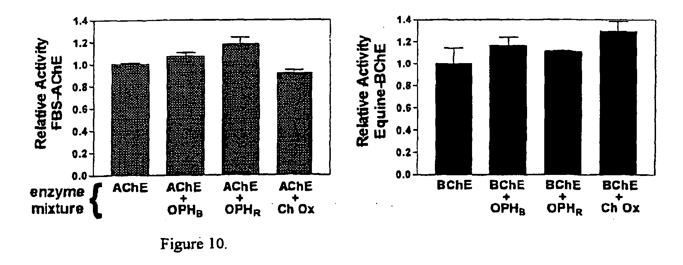

ChEs were co-immobilized with bacterial OP hydrolase (OPH_{B}) and/or rabbit serum OP hydrolase (OPH_{R}). There was no reduction in the enzymatic activities of AChE or BChE co-immobilized with OPH as compared to the enzymatic activities of each of these enzymes individually immobilized. *See* Figure 10. Additionally, there was no reduction in the enzymatic activity of co-immobilized OPH. Therefore, a plurality of enzymes, which each enzyme differentially reacts with various OP compounds, may be selected and utilized in a material to create a decontamination material effective against a wide range of OP compounds.

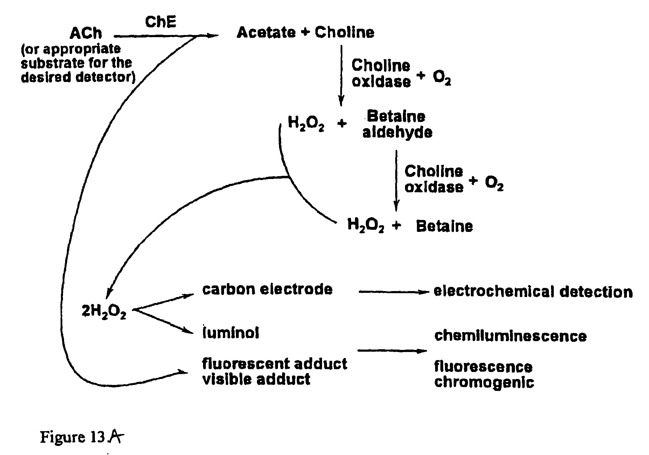

Lastly, an example of multiple enzymes required for alternate detection schemes (see Figure 13A) is also shown in Figure 10. The co-immobilization of choline oxidase (CH Ox) with AChE or BChE does not alter the immobilizing process or activity of the enzymes compared to the enzymes immobilized individually.

### Example 5

### Rapid Mixing Synthesis

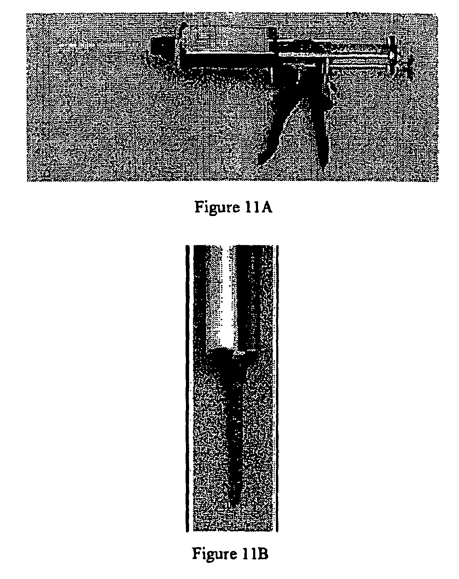

By utilizing a method of syntheses modified from the adhesive industry (CPA, Greenville, RI 02828) shear forces which decrease enzymatic activity are reduced. *See* Figure 11. In this method, the enzyme is not in an organic buffer as required in some immobilization techniques. This results in less air-induced shearing, thereby maintaining enzymatic activity. This method is also simple to conduct, rapid and reproducible. The low shear mixing device more than doubles the resultant AChE and/or BChE immobilized enzyme activity when compared to an identical mixture prepared with the high shear device such as a mixing drill. *See* Table 3.

| **Table 3** | |
|---|---|
| **Technique** | **AChE Activity U/mg** |
| High shear mixing drill | 0.100 |
| Low shear 2-chamber device | 0.270 |

### Example 6

### Determination of Enzymatic Activity

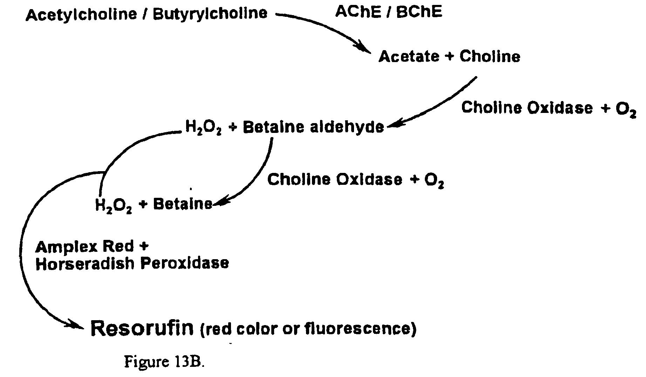

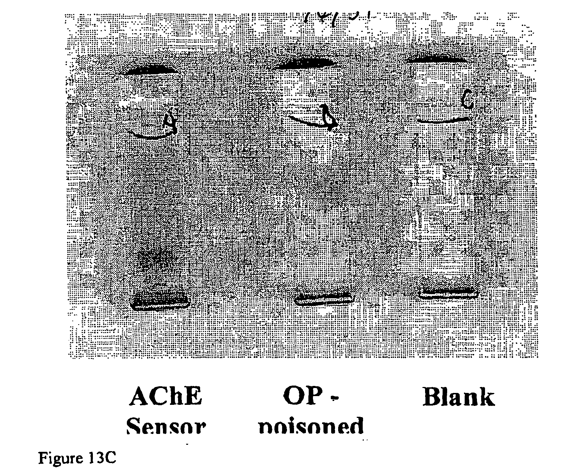

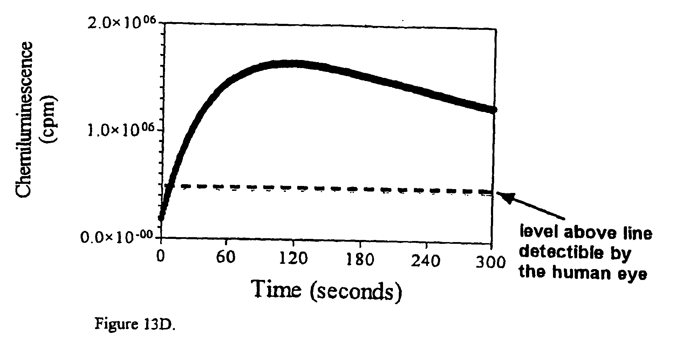

Figure 13 illustrates a variety of detection methods that may be employed with the biosensor of the present invention. Qualitative detection of the OP compounds may be visually performed by utilizing visible chromogens and/or chemiluminescent chromogens. Quantitative detection may be performed by using handheld devices, which measure amounts of fluorescence, chemiluminescence, or visible chromogens. In addition, an electronic biosensor comprising disposable carbon microelectrodes may be used to detect electrochemical signals from H₂O₂ generation.

The ChE activity of the materials were evaluated by using a modified Ellman method in an aqueous phosphate buffered environment containing either acetylthiocholine for AChE or butyrylthiocholine for BChE as substrates. The reactions, which produce an intense yellow color, were spectrophotometrically monitored at 412 nm. The final reaction product of the Ellman assay did not adsorb onto a material lacking ChE did not adsorb. The product generated was linear with time, thereby indicating that the release of the reaction product into the aqueous environment was not rate limiting. For determining the activity of the OP hydrolases, the substrate utilized was diethyl p-nitrophenylphosphate and the reactions were monitored at 500 nm.

The Ellman and OP hydrolase assays produce a yellow chromogen if the enzyme is not inhibited, and no color if the enzyme is inhibited. Alternatively, for cholinesterases, 2,6-dichloroindophenyl acetate (red color) turns blue (2,6 dichloroindophenylate) upon hydrolysis by ChE. Again, absence of a blue color indicates ChE inhibition by OP.

For fluorescent detection of ChE inhibition, the substrate may be either 1-methyl-7-acetoxyquinolinium iodide or fluorogenic maleimide *N*-(4-(7-diethylamino-4-methyl-coumarin-3-yl)phenyl)-maleimide. 1-methyl-7-acetoxyquinolinium iodide produces the highly fluorescent compound 1-methyl-7-hydroxyquinolinium iodide upon hydrolysis, i.e. 405nm/ex 505nm emission. Fluorogenic maleimide N-(4-(7-diethylamino-4-methyl-coumarin-3-yl)phenyl)-maleimide condenses with the thiol formed from acetyl- or butyrylthiocholine hydrolysis by ChEs, i.e. 390nm/ex 473nm emission.

For chemiluminescent detection, in addition to ChE and substrate (ACh or benzoylcholine), choline oxidase and peroxidase are added to a mixture containing luminol. In this chemiluminescent method, a light source is not needed, thereby allowing for a smaller, more energy efficient detectors. Additionally, chemiluminescent allows for detection with dark-adapted eyes. The indicator resorufin, a result of the reaction of Amplex Red (Molecular Probes, Inc.) and H₂O₂ has an advantage in that it is both flourescent, thereby yielding increased sensitivity, and is also a visible red chromogen (see Figure 13).

For the typical Ellman colorimetric assay, the lower limit for detecting OP compounds by standard equipment is approximately 35 femtograms of OP. This limit may be obtained with a one cm² piece of material wherein ChE is immobilized upon or within. This amount may be increased or decreased during synthesis of the material. Thus, the maximum cumulative amount of OP nerve agent which will covalently bind to the material is about 100 pg. The lowest immediate detectable level (<1 min) may be as low as 5 pg.

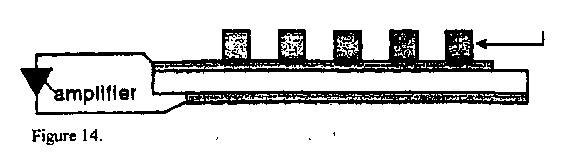

Microelectrodes may be used to detect ChE activity. Thus, the *in situ* cross-linking scheme outlined above may be used with multiple enzymes, e.g., ChEs, choline oxidase, and peroxidase. This mixture is then spotted and cured about a carbon electrode. *See* Figure 14. The enzyme-carbon electrode could be used to continuously analyze flowing liquids such as water without enzyme loss.

Optical analysis and common laboratory instrumentation such as UV-visible spectrophotometers, fluorometers, and electrochemical detectors may be used to develop a quantitative biosensor.

Similarly, the substrates and indicators above may be contained in small packets, liposomes or the like and embedded or incorporated within the porous support. When the material is squeezed, pressed or otherwise manipulated, the substrates and indicators are released and the resulting color change indicates the presence of an OP compound.

### Determination of Enzymatic Activity

A 10 mg sponge containing immobilized choline oxidase and immobilized AChE was incubated in a cuvette containing 2.5 mL of buffer containing substrate (acetylcholine), indicator (Amplex Red from Molecular Probes, Inc), and horseradish peroxidase. One sponge was exposed to MEPQ at 25°C for 5 min, rinsed in water and then placed in a second cuvette with the same buffer. Another cuvette contained only the above buffer but no biosensor sponge.

After a 5 min incubation at 25°C, the samples were observed visually, spectrophotometrically at 560 nm, and fluorometrically at 560 nm excitation and 580 nm emission. Visually against a white paper, the left sample (labeled A) demonstrates the intense color (red) observed when the sensor active and reacts with the substrate. The middle sample, labeled B, depicts the state of the sensor after being exposed to the OP MEPQ - it is poisoned and no longer produces color. Indeed, it is indistinguishable for the sample on the right, labeled C, which contains only the buffer and no sensor.

The same sample could be monitored fluorometrically or in the visual range using handheld spectrophotometers. The advantage of fluorometric analysis is the significant enhanced sensitivity over analysis in the visual spectrum. Using laboratory spectrophotometers, there was a 4000-fold enhancement of the signal and sensitivity in the cuvettes when measured fluorometrically.

The AChE and choline oxidase sensor can be used to indicate the absence or presence of organophosphates in complete darkness without an energy source. The AChE/choline oxidase immobilized sensor was incubated in 2.5 mL of buffer containing substrate (acetylcholine), luminol (5-amino-2,3-dihydro-1,4-phthalazinedione), 10 ug/mL horseradish peroxidase. Luminescence was quantified in a fluorometer without excitation (no light source) and emission at 0 order (wide open to measure light that would be generated by an unpoisoned sensor reaction). The AChE hydrolyzes the substrate and the choline oxidase acts on the product (see the scheme in Figure 13): the result is chemiluminescence. Chemiluminescence was observed for more than four minutes above the threshold of dark-adapted human eyes (indicated by the dotted horizontal line).

### Example 7

### Inhibition of Immobilized FBS-AChE with DFP

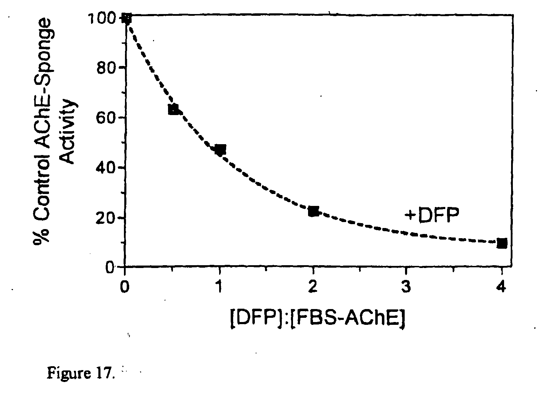

100 mg samples of immobilized FBS-AChE were incubated with varying concentrations of DFP in 2 mL of 50 mM phosphate buffer, pH 8.0, for 1 hour at 25°C. Residual DFP in the samples was measured by adding a 0.5 mL aliquot of the reaction mixture to 0.5 mL of a fresh 1 U/mL solution of FBS-AChE, incubating for 1 hour, and assaying 10 µl aliquots using the Ellman procedure. The results are shown in Figure 17.

The inhibition of FBS-AChE activity by DFP was proportional to the stoichiometric amount of DFP added to the foam suspended in buffer.

### Example 8

### Inhibition of Immobilized Eq-BChE with DFP

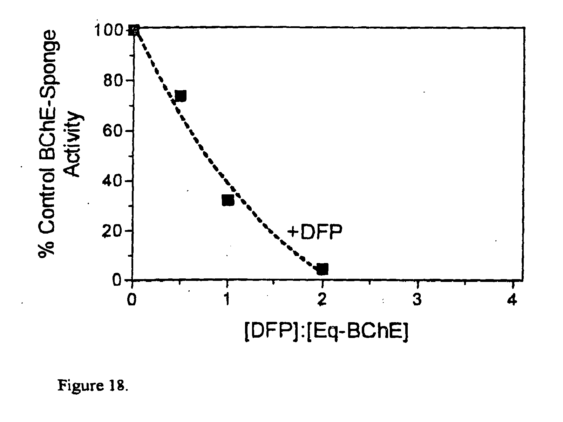

50 mg samples of immobilized Eq-BChE were incubated with varying concentrations of DFP in 2 mL of 50 mM phosphate buffer, pH 8.0, for 18 hours at 25°C. Residual DFP in the samples was determined by adding a 0.5 mL aliquot of the reaction mixture to 0.5 mL of a fresh 1 U/mL solution of Eq-BChE, incubating for 1 hour, and assaying 10 µl aliquots using the Ellman procedure. The results are shown in Figure 18.

### Example 9

### Differential Biosensor Comprising Multiple Immobilized Enzymes

Materials comprising cholinesterases, OP hydrolases, and enzymes which hydrolyze other OPs may be covalently immobilized upon or within a porous support to form a differential biosensor. A differential biosensor could be made of various ChEs, OP hydrolases and/or laccases immobilized on or within a porous support, wherein each ChE, OP hydrolase and/or laccase is individually and separately secured onto a carrier such as a piece of plastic. For example, serum OP hydrolase from rabbit exhibits high activity with sarin, but not with soman. Therefore, a single piece of plastic whereupon a material having immobilized OPHr and a material having OP hydrolase from a source other than rabbit serum are secured, could act as a biosensor which differentiates between sarin and soman.

Additionally, since the enzymes from several species of halophilic and *Alteromonas* bacteria have considerable variation in enzymatic activity towards organophosphorous compounds, a plurality of materials wherein these enzymes are immobilized on or within the porous support may be separately secured on a carrier for use as a differentially acting biosensor. For example, since OPH from *A: undi* displays higher enzymatic activity against soman with respect to sarin and/or tabun, OPH from *A. undi* and OPH from another source could act as a biosensor which differentially detects soman over sarin and/or tabun. Additionally, the various OP hydrolases, ChEs, laccases and/or mediators of laccases and mutations thereof may be screened or developed for exhibiting different characteristic specificities and/or activities towards the a plurality of OP compounds.

Table 4 outlines a few enzymes that may be utilized for a rapid quantitative and qualitative identification of given OP compounds. The carrier could be divided into several compartments containing separate spots of immobilized enzymes that show different specificity for each OP. For example, if an OP agent is present in the sample tested, there would be inhibition AChE and/or BChE, i.e., no color change in the AChE and/or BChE compartments. If the OP agent is sarin, rabbit OP hydrolase would hydrolyze sarin and result in a color change in the rabbit OP hydrolase compartment. Additionally, the compartment containing *A. undina* would undergo a modest color change as there is only modest inhibition of the applied substrate since sarin is only a partial substrate. If VX is present, the color of the compartment containing laccase would be altered as laccase hydrolyzes VX.

| **Table 4. Relative activity of enzyme** | | | | |
|---|---|---|---|---|
| OXYGEN PUMPING CELL | AChE or BChE | Rabbit OPH | *Alteromonas undi* | *Laccase* |
| Sarin | Inhibited | ++ | + | - |
| Soman | Inhibited | - | +++ | - |
| Tabun | Inhibited | - | + | - |
| VX | Inhibited | - | - | ++ |
| -, not tested or not hydrolyzed | | | | |

Thus, materials comprising immobilized enzymes such as those shown in Table 5 may be individually secured upon a carrier. After the carrier comprising the materials is exposed to the sample to be tested, the presence of particular OP agent may be determined by observing a color change of a given material in the presence of the appropriate substrate.

| **Table 5. Potential multiple immobilized enzymes** | | | |
|---|---|---|---|
| **Enzyme type and origin** | | **Distinguishing characteristics** | **References** |
| AChE, BChE | | Inhibited by OPs | 1, 2 |
| Laccase | | Hydrolyzes VX preferentially with mediator | 21 |
| OPH | Human serum | Hydrolyses tabun, VX poorly | 13 |
| | Rabbit serum | Hydrolyses sarin preferentially | 29, 32 |
| | *Pseudomonas* | Hydrolyses G agents | 33 |
| | *Alteromonas undi* | Hydrolyses soman preferentially | 17 |
| | Squid | Hydrolyses tabun, VX poorly | 34 |

### Example 10

### Remote Quantitative and Qualitative Analysis of OP Compound.

As an OP inhibited enzyme is not readily reversible and the enzyme is immobilized, the material may be transported from the test site to another site to be analyzed for the presence and amount of given OP compounds. Additionally, the material may be left at a site to monitor OP compounds for a period of time. Since the OP inhibited enzyme is not readily reversible, interfering compounds and compositions may be removed from the material either at the test site or at a different location. Furthermore, the analysis need not be conducted immediately or soon after sampling.

### A. Fluoride-Induced Release of OP

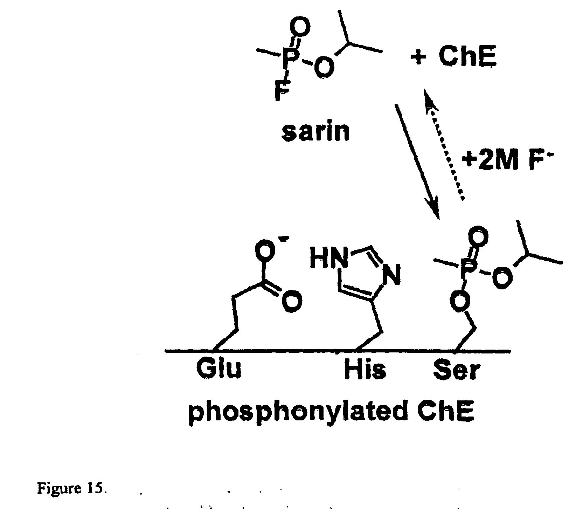

High concentrations of F⁻ cause the release of OP compound complexed to the inhibited ChE immobilized on the material. *See* Figure 15. This results in a soluble phosphofluoridate, which is specific for the OP compound present. The phosphofluoridate may be identified and quantified by gas chromatography and further verified with mass spectrometry in order to determine the original OP compound. Specifically, a material containing the inhibited ChE and washed free of interfering compounds is acidified to pH 4 and incubated with 2M potassium fluoride. The solution is then extracted with a C₁₈ SepPak (Waters Associates, Milford, Mass.). The OP compound is eluted and identified by gas chromatography and mass spectrometry. Most of the OP agents of interest may be identified and discriminated from OP pesticides. In this example, the samples need not be frozen in order to be tested for OP compounds at a later date since the material is extremely resistant to mechanical stress, harsh chemical conditions, and extreme and varying temperatures.

### B. Enzymatic Digestion

As an alternative procedure, enzymatic digestion may be used for post-exposure identification of OP compounds. The OP compounds may be released from the enzymes immobilized on or within the porous support and digested with 1M Tris buffer, pH 10, and alkaline phosphatase. Then the high molecular weight products may be concentrated, dissolved in a solution of pyridine and trimethylsilylation agents. The samples can then be analyzed by gas chromatography and mass spectrometry.

### Example 11

### Enzyme Coupling Prior to Formation of the Material.

The enzymes may be coupled together prior to formation of the material by means known in the art to form a cross-linked enzyme complex. *See e.g.* Hashida, S., Imagawa, M., Inoue, S., Ruan, K.-h, and Ishikawa, E. (1984) J. Applied Biochem. 6, 56-63 and Samaoszuk, M.K., Petersen, A., Lo-Hsueh, M., and Rietveld, C. (1989). (A peroxide-generating immunoconjugate directed to eosinophil peroxidase is cytotoxic to Hodgkin's disease cells in vitro.), Antibody Immunocon. Radiopharm. 2(1), 37-46.

For example, AChE may be conjugated to choline oxidase with one of the various cross-linkers and methods known in the art. Therefore, AChE and choline oxidase would be in close proximity so the product of AChE hydrolysis, choline, would fall right next to the choline oxidase to produce H₂O₂. This type of enzymatic cascade would provide more efficient coupling and a faster and more sensitive response. In addition, because of the proximity of choline oxidase, *i.e*. choline oxidase to AChE, the ratio of choline oxidase to AChE may be reduced. More than two different enzymes may be utilized.

The cross-linker utilized may be a multifunctional cross-linking agent. A wide variety of cross-linking agents are available from commercial suppliers, *i.e*. Pierce (Rockford, IL). These multifunctional cross-linking agents may comprise varying lengths of spacer arms to ensure that the bridge between the linked enzymes is an appropriate length for maintaining independent enzyme structure, function and activity. Typically, this would be a length of about 4-8 angstroms. However, the length may be up to 16 angstroms. Some cross-linking sites must be available for coupling the conjugated enzymes to the prepolymer. The cross-linking may be performed in the same buffer as used for the prepolymer reaction as explained in Example 2. The enzyme conjugate is then mixed with a prepolymer, as in Example 2, to form a polymeric material.

### Example 12

### Long-term Sensing of Aqueous environments for Organophosphates

A significant advantage of the immobilized enzymes is that they are covalent immobilized permanently within the polyurethane matrix. This affords the sensors with the following properties that are absent in the soluble state of the enzymes or when the enzymes are non-covalently attached to papers, tickets, or other indicating strips.

### A. Ability to Retain Activity after Continuous Incubation at 25°C at different pHs

The activity of immobilized AChE and BChE enzymes after 2 months at 25°C in buffers at pHs from 4.0 to 10.5 are shown in Figures 21A and 21B, respectively. Even after more than a month in solution without sterilization, both ChE sensors retained most of their original activity at pHs between 6-8, and significant activity was only lost at the extremes of pH4 and 10.5. The loss of activity at the extreme pHs is not unexpected since it is known that these conditions cause irreversible denaturation of the soluble enzymes. However, note that for short periods of less than a few days, 50% or more of the original activity of the immobilized enzymes remained, while the soluble enzyme would have been completely denatured. These results demonstrate that the ChEs are suitable for long-term (days to many weeks) detection of OPs. For instance, the sensor could be left at a remote location and retrieved at a later date.

### B. Ability to Retain Activity after Continuous Incubation in Natural Water Sources at Ambient (25°C) Temperatures

Additional evidence that the AChE sensor retains activity for extended periods in the environment is observed in Figure 21C (Exposure to Brackish Water, obtained from the Chesapeake Bay, Aberdeen, MD) and Figure 21D (Exposure to Fresh Water, obtained from the Allegheny River, PA). Most of the original activity of the sensor remains even when exposed to water for over 1 month. The immobilized enzyme was also resistant to natural microbiological flora and fauna that could degrade the enzyme since autoclaved water was not more stable than untreated water. Taken together, these results demonstrate the long-term sensing potential of these immobilized enzymes.

### C. Comparison of the M272 ticket with example A and B, above

The M272 ticket is the currently fielded ticket for sensing organophosphates in aqueous solutions. The ticket contains non-covalently bound Eel cholinesterase. In contrast to the 1-2 months that the immobilized AChE and BChE sensors can retain activity even after continuous exposure to natural water sources, varying pH, temperature (up to years), etc, the M272 ticket looses more than 80% of its activity after exposure to Chesapeake Bay water (Figure 21E) or a buffer (50 mM phosphate buffer, pH 8.0 (Figure 21F) after only 5 minutes of exposure. Therefore, while the immobilized enzymes are suitable for long-term monitoring of the environment including water, in contrast, the M272 ticket is not suitable for even short-term monitoring of water sources for organophosphorus compounds.

### Example 13

### Resistance to False Positives.

It is desirable to have a sensor resistant to false positives. In this definition, a false positive for OP would be any compound that denatures the enzyme. Since organic compounds are know to inhibit and denature many proteins and enzymes in their soluble form, the AChE sensor was evaluated for activity after exposure to saturated gasoline (Figure 21A) and diesel fumes (Figure 21B), conditions that could exist near fuel depots and machinery that are potential military or terrorist sites for attack with OPs. The immobilized enzymes retained more than 80% of their original activity after 2-3 hours of continuous exposure, and the polymer matrix showed no indication of breakdown. These results demonstrate that the matrix stabilized the immobilized acetylcholinesterase to denaturating conditions of the organic fuel vapors, thereby decreasing false positive responses.

### Example 14

### Sensitivity of Soluble and Immobilized Mammalian AChE to Pesticide (Dichlorophos) and Organophosphate (Soman, GD).

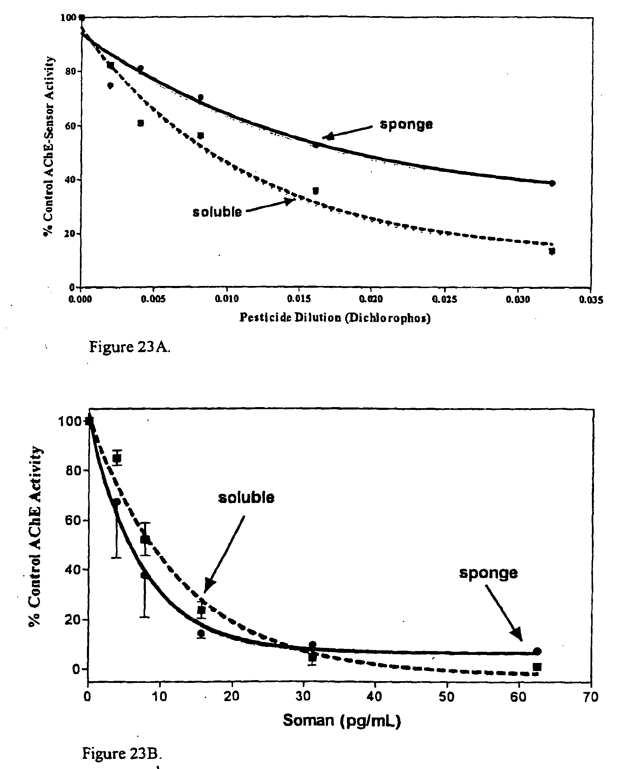

AChE sensor and soluble AChE were exposed to dilutions of Dichlorophos in 2.5 mL of 50 mM phosphate buffer for 5 minutes, and then the activity of the enzymes in soluble form and immobilized sensor were determined. As shown in Figure 23A, the sensitivity of the immobilized sensor and soluble enzyme exhibited very similar EC₅₀ values, however the slope for the sponge was about 20% less than the soluble enzyme. These results indicate that the AChE-sponge was slightly less sensitive to inhibition by the pesticide than the soluble mammalian enzyme.

Similar results were observed for the inhibition of AChE sensor (immobilized enzyme) and the soluble acetylcholinesterase. Figure 23B demonstrates that when the enzymes are exposed to soman for 5 minutes and then inhibition of the enzyme determined, the curves indicating loss of enzyme activity by soman exposure are not significantly different. Thus, in the absence of soman, there is color development and enzyme activity (100% level) while at 30 pg of soman, little color reaction develops and activity is less than 20% of the control level.

### References

Ashani, Y., Leader, H., Rothschild, N., and Dosoretz, C. Combined effect of organophosphorus hydrolase and oxime on the reactivation rate of diethylphosphoryl-acetylcholinesterase conjugates. Biochem. Pharmacol. 55:159-68 (1998).
Brenna, O., and E. Bianchi. Immobilised Laccase for Phenolic Removal in Must and Wine. Biotechnology Letters. 16:35-40 (1994).
DeFrank, J.J., Beaudry, W.T., Cheng, T-C., Harvey, S.P., Stroup, A.N., and Szafraniec, L.L. Screening of halophilic bacteria and *Alteromonas* species for organophosphorus hydrolyzing enzyme activity. Chem.-Biol. Interactions 87:141-148 (1993).
Donarski W.J., Dumas D.P., Heitmeyer D.P., Lewis V.E., Raushel, F.M. Structure-activity relationships in the hydrolysis of substrates by the phosphotriesterase from *Pseudomonas diminuta.* Biochemistry 28:4650-5 (1989).
Furlong, C.E., Richter, R.J., Chapline, C. and Crabb, J.W. Purification of rabbit and human serum paraoxonase. Biochemistry, 30:19133-10140 (1991).
Gan, K.N., Smolen, A., Eckerson, H.W., La Du, B.N. Purification of human serum paraoxonase/arylesterase. Evidence for one esterase catalyzing both activities. Drug. Metab. Disp. 19:100-106 (1991).
Hoskin, F.C., Roush, A.H. Hydrolysis of nerve gas by squid-type diisopropyl phosphorofluoridate hydrolyzing enzyme on agarose resin. Science 215:1255-7 (1982).
Maxwell, D.M., C.A. Castro, D.M. De La Hoz, M.K. Gentry, M.B. Gold, R.P. Solana, A.D. Wolfe, B.P. Doctor. Protection of rhesus monkeys against soman and prevention of performance decrement by treatment with acetylcholinesterase, Toxicol. Appl. Pharmacol. 115 44-49 91992).
Personal Communication, Dr. Gabriel Amitai, Israel Institute for Biological Research, Ness Ziona, Israel.
Taylor, P., Anticholinesterase agents, in: A.G. Gilman, T.W. Rall, A.S. Nies, P. Taylor (Eds.), The Pharmacological Basis of Therapeutics, Pergamon, New York, pp. 131-149 (1990).
Wang, F., Xiao, M., Mu, S. Purification and properties of a diisopropyl-fluorophosphatase from squid *Todarodes pacificus* Steenstrup. J. Biochem. Toxicol. 8:161-6 (1993).
Zouari, N., J.-L. Romette, and D. Thomas. Laccase Electrode for the continuous-Flow Determination of Phenolic Compounds. Biotechnology Techniques. 8:503-508 (1994).

## Claims

1. A biosensor for analyzing a sample for at least one organophosphorous and/or an organosulfur compound comprising at least one organophosphorus (OP) sensing enzyme and a measuring enzyme, which reacts with a product of OP sensing enzyme to form a detectable product, or a differential detection enzyme, which reacts with the organophosphorous or organosulfur compound to form a product which either now reacts with or does not react with the OP sensing enzyme, wherein the OP sensing enzyme is immobilized on or within a foam support by covalent bonds, wherein the OP sensing enzyme is selected from the group consisting of:
acetylcholinesterase (AChE), butyrylcholinesterase (BChE), and
pseudocholinesterase, the measuring enzyme is selected from choline oxidase or peroxidase and the differential detection enzyme is selected from triesterase, organophosphate hydrolase (OPH), laccase or paraoxonase and wherein the organophosphorous and/or organosulfur compounds are inhibitors of one or more of the OP sensing enzymes.

2. The biosensor of claim 1, which further comprises multiple zones wherein each zone contains a differential detection enzyme which differentiates between one or more of the organophosphorus and or organosulfur compound types.

3. The biosensor of claim 2, wherein the multiple zones form an array.

4. The biosensor of claim 1, wherein the biosensor is suitable for measuring or detecting organophosphorous and/or organosulfur compound(s) in gases or liquids or on solids.

5. The biosensor of claim 1 further comprising a carbon electrode for the detection of the organophosphorous and/or an organosulfur compound by measuring cholinesterase activity.

6. The biosensor of claim 1, wherein the foam is urethane.

7. The biosensor of claim 6, wherein the foam is synthesized from a reaction of a urethane, polyether or polyester polyol with isocyantates in the presence of crosslinking agents.

8. The biosensor of claim 7, wherein the foam is synthesized in the presence of the enzyme.

9. The biosensor of claim 8, wherein the enzyme is incorporated into the foam structure.

10. The biosensor of claim 7, wherein the synthesis is a rapid mixing one.

11. The biosensor of claim 1, wherein the foam support is secured upon a carrier.

12. The biosensor of claim 1, wherein the OP sensing enzyme, a first enzyme, is coimmobilized with or conjugated to an enzyme, a second enzyme, that reacts with a product produced by the first enzyme or with OP.

13. The biosensor of claim 12, wherein the first enzyme is a cholinesterase and the second enzyme is choline oxidase.

14. The biosensor of claim 12, wherein the first enzyme is a cholinesterase and the second enzyme is OP hydrolase.

15. A method for detecting and measuring at least one hazardous chemical in a sample, said chemical being an organophosphorus and/or an organosulfur compound which is an inhibitor of one or more of AChE, BChE and pseudocholinesterase, said method comprising contacting the biosensor of claim 1 with a sample and detecting and/or measuring the hazardous chemical.

16. The method of claim 15 further comprising applying an indicator for visually, chemically and/or electrically detecting and measuring a hazardous chemical wherein a visual, chemical and/or electrical change in the presence of said indicator indicates the presence and amount of the hazardous chemical.

17. The method of claim 15, wherein after the contacting step and before the detecting step the foam support is washed, squeezed or purified to remove compounds and/or compositions which may cause interference in the detecting step.

18. The method of claim 15, wherein the hazardous chemical is OP and the detecting and/or measuring occurs after the OP is released from the enzyme.

19. The method of claim 18, wherein the OP is released by contacting the enzyme with flourine anion and the detecting and measuring step involves the separation of OP and its measurement.

20. The method of claim 15, wherein the detection is based on the inhibition of cholinesterase activity.

21. The method of claim 15, wherein the presence of the enzyme is measured by its activity.

22. The method of claim 15, wherein activity is detected by an indicator for fluorescent, chemiluminescent or visible chromogen detection.

23. The method of claim 15, wherein the activity is determined based on an enzymatic substrate selected from acetylcholine, butyrylthiocholine, diethyl p-nitrophenylphosphate, 2, 6-dichloroindophenyl acetate, 1-methyl-7-acetoxyquinolinium iodide, n-(4-(7-diethylamino-4methyl-coumarin-3-yl)phenyl)-maleimide or other known substrates of cholinesterases.

24. A method of making a biosensor of claim 1 for detecting and measuring a hazardous compound said chemical being an organophosphorus and/or an organosulfur compound which is an inhibitor of one or more of AChE, BChE and pseudocholinesterase, said method comprising immobilizing by covalently binding a plurality of enzymes or a cross-linked enzyme complex on, within or encapsulated in a porous support wherein the plurality of enzymes or the cross-linked enzyme complex comprises at least one OP sensing enzyme and a measuring enzyme, which reacts with a product of OP sensing enzyme to form a detectable product, or a differential detection enzyme, which reacts with the organophosphorous or organosulfur compound to form a product which either now reacts with or does not react with the OP sensing enzyme, wherein the OP sensing enzyme is an enzyme selected from the group consisting of: acetylcholinesterase (AChE), butyrylcholinesterase (BChE), and pseudocholinesterase, the measuring enzyme is selected from choline oxidase or peroxidase and the differential detection enzyme is selected from triesterase, organophosphate hydrolase (OPH), laccase or paraoxonase.

25. The method of claim 24 wherein the step of immobilizing comprises mixing said plurality or said cross-linked enzyme complex with a polyurethane prepolymer.

26. The method of claim 25 wherein said polyurethane prepolymer comprises a mixture of at least one diisocyanate.

27. The method of claim 26 wherein the diisocyanate is tolyl diisocyanate.

28. The method of claim 25 wherein equal volume parts of said plurality or said cross-linked enzyme complex and said polyurethane prepolymer are simultaneously mixed under conditions which fold the plurality of enzymes or said cross-linked enzyme complex with the prepolymer.

29. The method of claim 27 wherein the mixing is done with a static mixing stator.

30. A kit for detecting and measuring at least one hazardous chemical in a sample comprising the biosensor of claim 1 and reagents for visually, chemically and/or electrically detecting and measuring the hazardous chemical, wherein the hazardous chemical is an organophosphorus and/or an organosulfur compound.

31. The kit of claim 30 further including means for transmitting results to a central collection point.

## Patentansprüche

1. Biosensor zur Analyse einer Probe bezüglich mindestens einer Organophosphor- und/oder einer Organoschwefelverbindung, der mindestens ein Organophosphor (OP)-erfassendes Enzym und ein Messenzym, das mit einem Produkt des OP-erfassenden Enzyms unter Bildung eines nachweisbaren Produkts reagiert, oder ein Differenzierungsnachweisenzym, das mit der Organophosphor- oder Organoschwefelverbindung unter Bildung eines Produkts reagiert, das mit dem OP-erfassenden Enzym reagiert oder nicht, umfasst, wobei das OP-erfassende Enzym mittels kovalenter Bindungen auf einem Schaumträger oder innerhalb eines Schaumträgers immobilisiert ist, wobei das OP-erfassende Enzym aus der Gruppe bestehend aus Acetylcholinesterase (AChE), Butyrylcholinesterase (BChE) und Pseudocholinesterase ausgewählt ist, wobei das Messenzym aus Cholinoxidase oder -peroxidase ausgewählt ist und wobei das Differenzierungsnachweisenzym aus Triesterase, Organophosphathydrolase (OPH), Laccase oder Paraoxonase ausgewählt ist, und wobei die Organophosphor- und/oder Organoschwefelverbindungen Inhibitoren von einem oder mehreren der OP-erfassenden Enzyme sind.

2. Biosensor nach Anspruch 1, der ferner mehrere Zonen umfasst, wobei jede Zone ein Differenzierungsnachweisenzym enthält, das zwischen einem oder mehreren der Typen von Organophosphor- und/oder Organoschwefelverbindungen differenziert.

3. Biosensor nach Anspruch 2, bei dem die mehreren Zonen einen Array bilden.

4. Biosensor nach Anspruch 1, wobei der Biosensor zum Messen oder Nachweisen von (einer) Organophosphor- und/oder Organoschwefelverbindung(en) in Gasen oder Flüssigkeiten oder auf Feststoffen geeignet ist.

5. Biosensor nach Anspruch 1, der ferner eine Kohlenstoffelektrode zum Nachweis einer Organophosphor- und/oder einer Organoschwefelverbindung durch Messen der Cholinesteraseaktivität umfasst.

6. Biosensor nach Anspruch 1, bei dem der Schaum ein Urethan ist.

7. Biosensor nach Anspruch 6, bei dem der Schaum durch eine Reaktion eines Urethans, eines Polyether- oder Polyesterpolyols mit Isocyanaten in der Gegenwart von Vernetzungsmitteln synthetisiert worden ist.

8. Biosensor nach Anspruch 7, bei dem der Schaum in der Gegenwart des Enzyms synthetisiert worden ist.

9. Biosensor nach Anspruch 8, bei dem das Enzym in die Schaumstruktur einbezogen ist.

10. Biosensor nach Anspruch 7, bei dem die Synthese eine Synthese mit schnellem Mischen ist.

11. Biosensor nach Anspruch 1, bei dem der Schaumträger auf einem Träger angebracht ist.

12. Biosensor nach Anspruch 1, bei dem das OP-erfassende Enzym, bei dem es sich um ein erstes Enzym handelt, mit einem Enzym coimmobilisiert ist oder an ein Enzym konjugiert ist, bei dem es sich um ein zweites Enzym handelt, das mit einem Produkt, das durch das erste Enzym erzeugt worden ist, oder mit OP reagiert.

13. Biosensor nach Anspruch 12, bei dem das erste Enzym eine Cholinesterase und das zweite Enzym Cholinoxidase ist.

14. Biosensor nach Anspruch 12, bei dem das erste Enzym eine Cholinesterase und das zweite Enzym OP-Hydrolase ist.

15. Verfahren zum Nachweisen und Messen mindestens einer gefährlichen Chemikalie in einer Probe, wobei die Chemikalie eine Organophosphor- und/oder eine Organoschwefelverbindung ist, bei der bzw. denen es sich um einen Inhibitor bzw. Inhibitoren von einem oder mehreren von AChE, BChE und Pseudocholinesterase handelt, wobei das Verfahren das In-Kontakt-Bringen des Biosensors von Anspruch 1 mit einer Probe und das Nachweisen und/oder Messen der gefährlichen Chemikalie umfasst.

16. Verfahren nach Anspruch 15, das ferner das Aufbringen eines Indikators zum visuellen, chemischen und/oder elektrischen Nachweisen und Messen einer gefährlichen Chemikalie umfasst, wobei eine visuelle, chemische und/oder elektrische Veränderung in der Gegenwart des Indikators die Gegenwart und die Menge der gefährlichen Chemikalie angibt.

17. Verfahren nach Anspruch 15, bei dem nach dem Schritt des In-Kontakt-Bringens und vor dem Nachweisschritt der Schaum gewaschen, ausgedrückt oder gereinigt wird, um Verbindungen und/oder Zusammensetzungen zu entfernen, die eine Störung in dem Nachweisschritt verursachen können.

18. Verfahren nach Anspruch 15, bei dem die gefährliche Chemikalie eine OP ist und der Nachweis und/oder die Messung stattfindet bzw. stattfinden, nachdem die OP von dem Enzym freigesetzt worden ist.

19. Verfahren nach Anspruch 18, bei dem die OP durch In-Kontakt-Bringen des Enzyms mit einem Fluoranion freigesetzt wird und der Nachweis- und Messschritt die Abtrennung der OP und deren Messung umfasst.

20. Verfahren nach Anspruch 15, bei dem der Nachweis auf der Inhibierung der Cholinesteraseaktivität beruht.

21. Verfahren nach Anspruch 15, bei dem die Gegenwart des Enzyms durch dessen Aktivität gemessen wird.

22. Verfahren nach Anspruch 15, bei dem die Aktivität durch einen Indikator für einen fluoreszierenden, chemilumineszierenden oder sichtbaren Chromogennachweis nachgewiesen wird.

23. Verfahren nach Anspruch 15, bei dem die Aktivität auf der Basis eines enzymatischen Substrats bestimmt wird, das aus Acetylcholin, Butyrylthiocholin, Diethyl-p-nitrophenylphosphat, 2,6-Dichlorindophenylacetat, 1-Methyl-7-acetoxychinoliniumiodid, n-(4-(7-Diethylamino-4-methylcumarin-3-yl)phenyl)maleimid oder anderen bekannten Substraten von Cholinesterasen ausgewählt ist.

24. Verfahren zur Herstellung eines Biosensors nach Anspruch 1 zum Nachweisen und Messen einer gefährlichen Verbindung, wobei die Chemikalie eine Organophosphor- und/oder eine Organoschwefelverbindung ist, bei der bzw. denen es sich um einen Inhibitor bzw. um Inhibitoren von einem oder mehreren von AChE, BChE und Pseudocholinesterase handelt, wobei das Verfahren das Immobilisieren durch kovalentes Binden einer Mehrzahl von Enzymen oder eines vernetzten Enzymkomplexes auf einem porösen Träger, innerhalb eines porösen Trägers oder eingekapselt in einen porösen Träger umfasst, wobei die Mehrzahl von Enzymen oder der vernetzte Enzymkomplex mindestens ein OP-erfassendes Enzym und ein Messenzym, das mit einem Produkt des OP-erfassenden Enzyms unter Bildung eines nachweisbaren Produkts reagiert, oder ein Differenzierungsnachweisenzym, das mit der Organophosphor- oder Organoschwefelverbindung unter Bildung eines Produkts reagiert, das mit dem OP-erfassenden Enzym reagiert oder nicht, umfasst, wobei das OP-erfassende Enzym ein Enzym ist, das aus der Gruppe bestehend aus Acetylcholinesterase (AChE), Butyrylcholinesterase (BChE) und Pseudocholinesterase ausgewählt ist, wobei das Messenzym aus Cholinoxidase oder -peroxidase ausgewählt ist und wobei das Differenzierungsnachweisenzym aus Triesterase, Organophosphathydrolase (OPH), Laccase oder Paraoxonase ausgewählt ist.

25. Verfahren nach Anspruch 24, bei dem der Schritt des Immobilisierens das Mischen der Mehrzahl von Enzymen oder des vernetzten Enzymkomplexes mit einem Polyurethan-Vorpolymer umfasst.

26. Verfahren nach Anspruch 25, bei dem das Polyurethan-Vorpolymer ein Gemisch aus mindestens einem Diisocyanat umfasst.

27. Verfahren nach Anspruch 26, bei dem das Diisocyanat Tolyldiisocyanat ist.

28. Verfahren nach Anspruch 25, bei dem gleiche Volumenteile der Mehrzahl der Enzyme oder des vernetzten Enzymkomplexes und des Polyurethan-Vorpolymers gleichzeitig unter Bedingungen gemischt werden, welche die Mehrzahl von Enzymen oder den vernetzten Enzymkomplex mit dem Vorpolymer vereinigen.

29. Verfahren nach Anspruch 27, bei dem das Mischen mit einem statischen Mischstator durchgeführt wird.

30. Kit zum Nachweisen und Messen mindestens einer gefährlichen Chemikalie in einer Probe, das den Biosensor nach Anspruch 1 und Reagenzien zum visuellen, chemischen und/oder elektrischen Nachweisen und Messen der gefährlichen Chemikalie umfasst, wobei die gefährliche Chemikalie eine Organophosphor- und/oder eine Organoschwefelverbindung ist.

31. Kit nach Anspruch 30, das ferner Mittel zum Übertragen von Ergebnissen an einen zentralen Sammelpunkt umfasst.

## Revendications

1. Biodétecteur pour analyser un échantillon pour au moins un composé organophosphoré et/ou organosulfuré qui comprend au moins une enzyme de détection d'organophosphoré (OP) et une enzyme de mesure, qui réagit avec un produit de l'enzyme de détection d'OP pour former un produit pouvant être détecté, ou une enzyme de détection différentielle, qui réagit avec le composé organophosphoré ou organosulfuré pour former un produit qui soit réagit alors avec ou ne réagit pas avec l'enzyme de détection d'OP, dans lequel l'enzyme de détection d'OP est immobilisée sur ou à l'intérieur d'un support en mousse par des liaisons covalentes, dans lequel l'enzyme de détection d'OP est choisie dans le groupe constitué de :
acétylcholinestérase (AChE), butyrylcholinestérase (BChE), et pseudocholinestérase, l'enzyme de mesure est choisie parmi les choline oxydase ou peroxydase et l'enzyme de détection différentielle est choisie parmi la triestérase, l'organophosphate hydrolase (OPH), la laccase ou la paraoxonase et dans lequel les composés organophosphorés et/ou organosulfurés sont des inhibiteurs d'une ou plusieurs des enzymes de détection d'OP.

2. Biodétecteur selon la revendication 1, qui comprend en outre de multiples zones dans lequel chaque zone contient une enzyme de détection différentielle qui différencie entre un ou plusieurs des types de composés organophosphorés et/ou organosulfurés.

3. Biodétecteur selon la revendication 2, dans lequel les zones multiples forment un réseau.

4. Biodétecteur selon la revendication 1, dans lequel le biodétecteur convient à la mesure ou à la détection de composé(s) organophosphoré(s) et/ou organosulfuré(s) dans des gaz ou des liquides ou sur des solides.

5. Biodétecteur selon la revendication 1 qui comprend en outre une électrode de carbone pour la détection du composé organophosphoré et/ou d'un organosulfuré en mesurant l'activité de la cholinestérase.

6. Biodétecteur selon la revendication 1, dans lequel la mousse est un uréthanne.

7. Biodétecteur selon la revendication 6, dans lequel la mousse est synthétisée à partir d'une réaction d'un polyol d'uréthanne, de polyéther ou de polyester avec des isocyanates en présence d'agents de réticulation.

8. Biodétecteur selon la revendication 7, dans lequel la mousse est synthétisée en présence de l'enzyme.

9. Biodétecteur selon la revendication 8, dans lequel l'enzyme est incorporée à l'intérieur de la structure de la mousse.

10. Biodétecteur selon la revendication 7, dans lequel la synthèse est une synthèse par mélange rapide.

11. Biodétecteur selon la revendication 1, dans lequel le support en mousse est fixé sur un support.

12. Biodétecteur selon la revendication 1, dans lequel l'enzyme de détection d'OP, une première enzyme, est immobilisée conjointement avec ou conjuguée à une enzyme, une seconde enzyme, qui réagit avec un produit produit par la première enzyme ou avec l'OP.

13. Biodétecteur selon la revendication 12, dans lequel la première enzyme est une cholinestérase et la seconde enzyme est la choline oxydase.

14. Biodétecteur selon la revendication 12, dans lequel la première enzyme est une cholinestérase et la seconde enzyme est la OP hydrolase.

15. Procédé pour détecter et mesurer au moins un produit chimique dangereux dans un échantillon, ledit produit chimique étant un composé organophosphoré et/ou organosulfuré qui est un inhibiteur d'une ou plusieurs des AChE, BChE et pseudocholinestérase, ledit procédé comprenant la mise en contact du biodétecteur selon la revendication 1 avec un échantillon et la détection et/ou la mesure du produit chimique dangereux.

16. Procédé selon la revendication 15 qui comprend en outre l'application d'un indicateur de détection et de mesure visuelle, chimique et/ou électrique d'un produit chimique dangereux dans lequel un changement visuel, chimique et/ou électrique en présence dudit indicateur indique la présence et la quantité de produit chimique dangereux.

17. Procédé selon la revendication 15, dans lequel après l'étape de mise en contact et avant l'étape de détection le support en mousse est lavé, pressé ou purifié pour enlever des composés et/ou des compositions qui peuvent causer une interférence dans l'étape de détection.

18. Procédé selon la revendication 15, dans lequel le produit chimique dangereux est l'OP et la détection et/ou la mesure se produit après que l'OP est libéré de l'enzyme.

19. Procédé selon la revendication 18, dans lequel l'OP est libéré par la mise en contact de l'enzyme avec un anion fluor et l'étape de détection et de mesure implique la séparation de l'OP et sa mesure.

20. Procédé selon la revendication 15, dans lequel la détection est basée sur l'inhibition de l'activité de la cholinestérase.

21. Procédé selon la revendication 15, dans lequel la présence de l'enzyme est mesurée par son activité.

22. Procédé selon la revendication 15, dans lequel l'activité est détectée par un indicateur pour la détection fluorescente, chimioluminescente ou chromogène visible.

23. Procédé selon la revendication 15, dans lequel l'activité est déterminée en se basant sur un substrat enzymatique choisi parmi les acétylcholine, butyrylthiocholine, p-nitrophénylphosphate de diéthyle, acétate de 2,6-dichloroindophényle, iodure de 1-méthyl-7-acétoxyquinolinium, n-(4-(7-diéthylamino-4méthyl-coumarin-3-yl)phényl)maléimide ou d'autres substrats connus de cholinestérases.

24. Procédé de fabrication d'un biodétecteur selon la revendication 1 pour détecter et mesurer un composé chimique dangereux, ledit produit chimique étant un composé organophosphoré et/ou organosulfuré qui est un inhibiteur d'une ou plusieurs des AChE, BChE et pseudocholinestérase, ledit procédé comprenant l'immobilisation d'une pluralité d'enzymes ou d'un complexe enzymatique réticulé en les liant de manière covalente sur, à l'intérieur ou encapsulés dans un support poreux dans lequel la pluralité d'enzymes ou le complexe enzymatique réticulé comprend au moins une enzyme de détection d'OP et une enzyme de mesure, qui réagit avec un produit de l'enzyme de détection d'OP pour former un produit pouvant être détecté, ou une enzyme de détection différentielle, qui réagit avec le composé organophosphoré ou organosulfuré pour former un produit qui soit réagit alors avec ou ne réagit pas avec l'enzyme de détection d'OP, dans lequel l'enzyme de détection d'OP est une enzyme choisie dans le groupe constitué de : l'acétylcholinestérase (AChE), la butyrylcholinestérase (BChE), et la pseudocholinestérase, l'enzyme de mesure est choisie parmi la choline oxydase ou peroxydase et l'enzyme de détection différentielle est choisie parmi la triestérase, l'organophosphate hydrolase (OPH), la laccase ou la paraoxonase.

25. Procédé selon la revendication 24, dans lequel l'étape d'immobilisation comprend le mélange de ladite pluralité ou dudit complexe enzymatique réticulé avec un prépolymère polyuréthanne.

26. Procédé selon la revendication 25, dans lequel ledit prépolymère polyuréthane comprend un mélange d'au moins un diisocyanate.

27. Procédé selon la revendication 26, dans lequel le diisocyanate est le tolyl diisocyanate.

28. Procédé selon la revendication 25, dans lequel des parties en volume égales de ladite pluralité ou dudit complexe enzymatique réticulé et dudit prépolymère polyuréthanne sont simultanément mélangées dans des conditions qui incorporent la pluralité d'enzymes ou ledit complexe enzymatique réticulé avec le prépolymère.

29. Procédé selon la revendication 27, dans lequel le mélange est effectué avec un stator mélangeur statique.

30. Kit pour détecter et mesurer au moins un produit chimique dangereux dans un échantillon comprenant le biodétecteur selon la revendication 1 et des réactifs pour détecter et mesurer visuellement, chimiquement et/ou électriquement le produit chimique dangereux, dans lequel le produit chimique dangereux est un composé organophosphoré et/ou organosulfuré.

31. Kit selon la revendication 30, qui comprend en outre des moyens pour transmettre des résultats à un point de collecte central.
